(19) Europäisches Patentamt
European Patent Office
Office européen des brevets

(11) Numéro de publication: **0 201 378 B1**

(12) # FASCICULE DE BREVET EUROPEEN

(45) Date de publication de fascicule du brevet: **27.11.91**

(51) Int. Cl.5: **C08F 8/32**, C08J 7/14, A61M 25/00, B01D 61/00, B01D 15/08

(21) Numéro de dépôt: **86400737.2**

(22) Date de dépôt: **07.04.86**

(54) **Objets insolubles à usage médical ou chirurgical tels que membranes de dialyse rénale ou de plasmaphérèse, sondes, supports pour épuration plasmatique, ayant une activité anti-inflammatoire.**

(30) Priorité: **11.04.85 FR 8505462**

(43) Date de publication de la demande: **17.12.86 Bulletin 86/46**

(45) Mention de la délivrance du brevet: **27.11.91 Bulletin 91/48**

(84) Etats contractants désignés: **BE CH DE GB IT LI NL SE**

(56) Documents cités:
**EP-A- 0 023 854**
**US-A- 2 911 378**
**US-A- 3 337 480**

(73) Titulaire: **CIS BIO INTERNATIONAL**
**RN 306**
**F-91000 Saclay(FR)**

(72) Inventeur: **Carreno, Marie-Paule**
**6, Allée des Pierrats**
**F-95870 Bezons(FR)**
Inventeur: **Josefonvicz, Jacqueline**
**65, Deuxième Avenue**
**F-60260 Lamorlaye(FR)**
Inventeur: **Jozefowicz, Marcel**
**65, Deuxième Avenue**
**F-60260 Lamorlaye(FR)**
Inventeur: **Kazatchkine, Micel**
**20, rue du Commandant Mouchotte**
**F-75014 Paris(FR)**
Inventeur: **Labarre, Denis**
**103, Les Eaux Vives**
**F-91120 Palaiseau(FR)**

(74) Mandataire: **Pottier, Pierre Société BREVATOME et al**
**25, Rue de Ponthieu**
**F-75008 Paris(FR)**

EP 0 201 378 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

**Description**

La présente invention concerne des objets insolubles à usage médical et/ou chirurgical, ayant une activité anti-inflammatoire.

Les réactions inflammatoires de l'organisme correspondent à un ensemble de phénomènes ayant pour but de défendre l'organisme contre l'intrusion de substances étrangères. Ces phénomènes mettent en jeu un grand nombre de mécanismes tant humoraux que cellulaires qui conduisent soit à la cicatrisation, soit à une inflammation chronique pathologique. L'inflammation peut être déclenchée aussi bien par des lésions physiques des tissus, des réactions chimiques, l'introduction de germes ou de divers antigènes. Les amorceurs physiologiques de l'inflammation sont des protéines qui, sous l'action des stimulis précités, activent et déclenchent différents mécanismes parmi lesquels on trouve en particulier le mécanisme d'activation du système du complément conduisant à la lyse cellulaire.

Cette activation du système du complément peut être amorcée par des anticorps du type IgG ou IgM liés à un antigène. Elle peut être aussi déclenchée en l'absence d'anticorps par contact des protéines du système du complément avec des surfaces activatrices telles que des surfaces en polysaccharide.

L'inflammation aiguë est une réaction normale qui doit se produire pour conduire à la guérison. Toutefois, dans le cas où l'on est amené à mettre en contact avec un organisme vivant une surface étrangère en vue d'un traitement local, ce qui est le cas par exemple avec les appareils d'hémodialyse et de plasmaphérèse, les sondes, les implants, les poches de stomie, etc..., il serait intéressant de pouvoir inhiber l'activation du système du complément induite par ces surfaces afin de réduire la réponse inflammatoire de l'organisme et de pouvoir poursuivre le traitement dans de bonnes conditions. C'est le cas en particulier lors de la pose de sondes qui engendrent une réponse inflammatoire au niveau des passages transcutanés en raison des lésions épithéliales. Il en est de même lors des traitements d'épuration ex vivo d'un liquide biologique qui peuvent déclencher à distance une activation du système du complément et conduire à des accidents, par exemple dans le cas de dialyse rénale ou d'épurateur plasmatique.

Aussi, des recherches ont été conduites pour découvrir de nouveaux biomatériaux utilisables pour la réalisation d'objets à usage médical et/ou chirurgical ne provoquant pas l'activation du système du complément ou inhibant l'activation de ce système lorsque celui-ci a été activé par d'autres moyens.

Ces dernières années, on a mis au point de nouveaux biomatériaux présentant des propriétés intéressantes pour une utilisation en chirurgie et en médecine, en partant de polymères sur lesquels on fixe des substituants particuliers ayant une activité biologique.

Ainsi, le document US-A- 2 911 378 décrit des copolymères styrène-divinyl benzène comportant des groupements

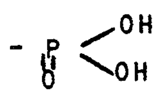

qui peuvent être utilisés en médecine sous la forme de leurs sels de sodium et de potassium comme matériau échangeur d'ion pour appauvrir en sel le corps humain. Il ne s'agit donc pas dans ce cas de l'activité anti-complémentaire recherchée.

Le brevet européen EP-A-0 023 854 décrit des polymères ayant des propriétés anticoagulantes qui sont dues à la fixation sur la chaîne du polymère de groupes X et/ou Y et/ou V, où :

- X désigne le groupe $-SO_3R_1$ ou $-R_3SO_3R_1$ avec $R_1$ étant un atome d'hydrogène ou d'un métal physiologiquement compatible, et $R_3$ étant un groupe $-CH_2-CO-NH-R_4$ dans lequel $R_4$ représente un radical alkyle, aryle ou alkylaryle substitué ou non ou le groupe

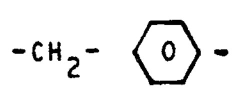

substitué ou non,
- Y désigne le groupe $-SO_2-R_2$ ou $-R_3-SO_2-R_2$ avec $R_2$ étant le reste d'un acide aminé lié au pont $-SO_2-$ par sa fonction amine, et
- V désigne le groupe $-CH_2-CO-NH-CHR-COOH$ avec R étant la chaîne latérale d'un acide aminé.

Les polymères utilisés peuvent être des polystyrènes ou des polysaccharides, par exemple des dextranes. Ces biomatériaux présentent une activité anti-coagulante grâce à la présence des groupes X

et/ou Y et/ou V. Cependant, lorsque X est $-SO_3R_1$, l'activité anti-coagulante ne se manifeste que si le polymère comporte simultanément des groupes Y et/ou V. De même, lorsque les groupes fixés sur le polymère sont des groupes V, l'activité anticoagulante n'est observée que lorsque le polymère comprend également des groupes X et/ou Y.

A la suite de ces travaux, on a recherché des biomatériaux à base de polymères ou de copolymères comportant des substituants capables de conférer à ces polymères la propriété de ne pas activer le système du complément ou d'inhiber l'activation de ce système lorsque celui-ci a été activé par d'autres moyens.

A la suite de ces recherches, on a trouvé que les substituants utilisés dans le brevet européen EP-0 023 854 avaient également la propriété d'inhiber l'activation du système du complément et que de plus des groupes tels que $-SO_3R_1$ ou V pouvaient conférer cette propriété au polymère sans être accompagnés d'autres groupes tels que Y et/ou V dans le cas de $SO_3R_1$ et tels que X et/ou Y dans le cas de V.

La présente invention a ainsi pour but de fournir des objets insolubles à usage médical et/ou chirurgical ayant une activité anti-inflammatoire, réalisés en polymères comportant des substituants particuliers.

Selon l'invention, l'objet insoluble à usage médical et/ou chirurgical se caractérise en ce qu'il est réalisé au moins en partie en un polymère ou un copolymère comportant dans sa chaîne des groupes substituables sur lesquels sont fixés de façon statistique des groupes inhibiteurs de l'activation du système du complément choisis parmi les groupes répondant aux formules suivantes :

1) - $-(CH_2)_mCOOR^1$, dans laquelle $R^1$ représente un atome d'hydrogène ou d'un métal physiologiquement acceptable et m est égal à 0 ou est un nombre entier de 1 à 15 ;

2) - $-CH_2-CO-NH-X$, dans laquelle X représente $-R^2-H$ où $R^2$ représente

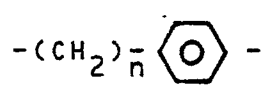

avec n étant un nombre entier de 1 à 4 ou un radical alkylène, arylène ou alkylènearylène substitué ou non ;

3) - $-(CH_2)_mCOY$, dans laquelle Y représente un radical dérivé d'un acide aminé ou d'un sel d'acide aminé lié au pont -CO- par sa fonction amine, et m est égal à 0 ou est un nombre entier de 1 à 15,

4) - $-SO_3R^1$, dans laquelle $R^1$ a la signification donnée ci-dessus,

5) -

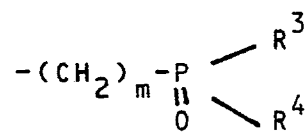

dans laquelle $R^3$ et $R^4$ qui peuvent être identiqes ou différents, représentent $OR^1$ ou Y avec $R^1$ ayant la signification donnée ci-dessus et Y représentant un radical dérivé d'un acide aminé lié au pont -PO- par sa fonction amine, et m est égal à 0 ou est un nombre entier allant de 1 à 15, à condition que $R^3$ et $R^4$ représentent OH ou Y lorsque m est égal à 0, et

6) -

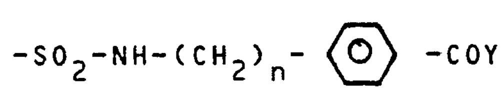

dans laquelle Y représente un radical dérivé d'un acide aminé lié au pont CO par sa fonction amine et n est un nombre entier de 1 à 4 ; à condition que le polymère ou le copolymère ne comporte qu'un seul type de groupes inhibiteurs lorsque ces groupes ont pour formule $-SO_3R_1$ ou $-(CH_2)_mCOY$ avec m étant égal à 1.

Les polymères et copolymères utilisés dans l'invention peuvent être des activateurs du système du complément. Dans ce cas, la fixation des groupes inhibiteurs de l'activation du système du complément mentionnés ci-dessus permet de rendre ces polymères et copolymères non activateurs du système du complément.

L'invention s'applique également à des polymères et copolymères non activateurs du système du

complément. Dans ce cas, la fixation des groupes inhibiteurs mentionnés ci-dessus rend ces polymère inhibiteurs de l'activation du système du complément induite par d'autres agents.

Les polymères et copolymères utilisés dans l'invention peuvent être de différents types. On peut en particulier utiliser des polymères tels que des polysaccharides, des polystyrènes, des polyacrylonitriles, des polypropylènes, des polymères de cellulose ou de dérivés de cellulose, des polyacétates de vinyle, des alcools polyvinyliques acétylés et des polysulfones aromatiques. On peut aussi utiliser des mélanges de polymères etdes copolymères par exemple des copolymères d'acrylonitrile comme le copolymère acrylonitrile-butadiène-styrène.

Parmi les polymères susceptibles d'être utilisés dans l'invention, on peut citer les polysaccharides, par exemple les dextranes et la cellulose, et les polystyrènes.

Selon l'invention, on peut aussi utiliser d'autres groupes inhibiteurs de l'activation du système du complément, en particulier certains des groupes connus pour posséder des propriétés anti-coagulantes.

Aussi, l'invention a également pour objet l'utilisation, pour la réalisation d'objets à usage médical ou chirurgical ayant une activité anti-complémentaire, de polymères ou copolymères comportant dans leur chaîne des groupes substituables sur lesquels sont fixés de façon statistique des groupes inhibiteurs de l'activation du système du complément choisis parmi les groupes répondant aux formules suivantes :

1) - $-(CH_2)_mCOOR^1$, dans laquelle $R^1$ représente un atome d'hydrogène ou d'un métal physiologiquement acceptable et m est égal à 0 ou est un nombre entier de 1 à 15 ;

2) - $-CH_2$-CO-NH-X, dans laquelle X représente $R^2$-$(SO_3)_pR^1$ avec p étant égal à 0 ou 1 et $R^1$ représentant un atome d'hydrogène ou d'un métal physiologiquement acceptable à condition que $R^1$ représente un atome d'hydrogène lorsque p est égal à 0, et $R^2$ représentant

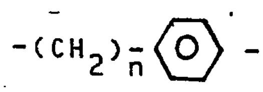

avec n étant un nombre entier de 1 à 4 ou un radical alkylène, arylène ou alkylènearylène substitué ou non ;

3) - $-CH_2$-CO-NH-$R^2$-$SO_2$-Y, dans laquelle $R^2$ représente

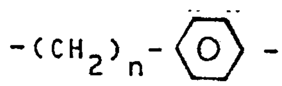

avec n étant un nombre entier de 1 à 4 ou un radical alkylène, arylène ou alkylènearylène substitué ou non, et Y représente un radical dérivé d'un acide aminé ou d'un sel d'acide aminé lié au pont $-SO_2$- par sa fonction amine ;

4) - $-SO_2Y$ dans laquelle Y a la signification donnée ci-dessus,

5) - $-(CH_2)_mCOY$, dans laquelle Y représente un radical dérivé d'un acide aminé lié au pont -CO- par sa fonction amine, et m est égal à 0 ou est un nombre entier de 1 à 15,

6) - $-SO_3R^1$, dans laquelle $R^1$ a la signification donnée ci-dessus,

7) -

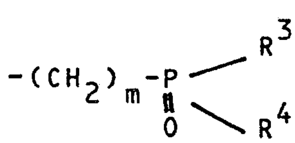

dans laquelle $R^3$ et $R^4$ qui peuvent être identiques ou différents, représentent $OR^1$ ou Y avec $R^1$ ayant la signification donnée ci-dessus et Y représentant un radical dérivé d'un acide aminé lié au pont -PO- par sa fonction amine, et m est égal à 0 ou est un nombre entier allant de 1 à 15, et

8) -

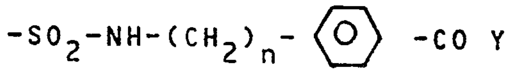

dans laquelle Y représente un radical dérivé d'un acide animé lié au pont CO par la fonction amine et n est un nombre entier de 1 à 4.

Dans le cas des polysaccharides qui sont généralement activateurs du système du complément, la fixation des groupes mentionnés ci-dessus permet de rendre ceux-ci non activateurs du système du complément, ce qui présente notamment un intérêt pour la réalisation des membranes de dialyse ou de plasmaphérèse qui généralement sont réalisées en de tels polymères.

Dans le cas des polysaccharides, les groupes inhibiteurs fixés sur le polymère sont généralement choisis parmi $-CH_2COOR^1$, $-CH_2-CO-Y$ et $-CH_2-CO-NH-X$.

Dans ces formules, $R^1$ peut représenter un atome d'hydrogène ou d'un métal physiologiquement acceptable, par exemple de sodium, Y peut représenter un radical dérivé d'un acide aminé naturel ou synthétique ou d'un sel de cet acide aminé, par exemple d'un sel de sodium, lié au pont CO par sa fonction amine.

A titre d'exemples d'acides aminés susceptibles d'être utilisés, on peut citer l'acide glutamique, l'acide aspartique, la méthionine, la cystéine, l'acide cystéique, la proline, l'hydroxyproline, la thréonine, la sérine, la tyrosine, l'alanine, la phénylalanine, la valine, la leucine, l'acide $\epsilon$-aminocaproïque, la $\beta$-alanine, l'acide $\gamma$-amino-N-butyrique, l'acide $\delta$-amino-N-valérique, l'acide 2 aminoadipique.

Y peut aussi représenter un radical dérivé d'un acide aminé répondant aux formules suivantes :

1°)

1°) 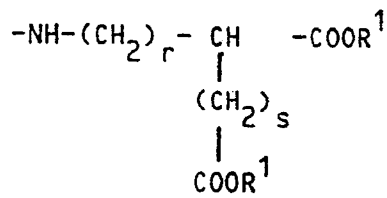

avec r étant égal à 0 ou étant un nombre entier de 1 à 3, s étant un nombre entier de 1 à 3 et $R^1$ ayant la signification donnée ci-dessus ; ou

2°) $NH-(CH_2)_t-COOR^1$ avec t étant un nombre entier de 1 à 15 et $R^1$ ayant la signification donnée ci-dessus.

Dans la formule donnée plus haut, X peut représenter un radical de formule :

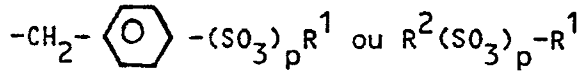

dans lesquelles $p=0$ ou 1, $R^1$ représente comme précédemment un atome d'hydrogène ou d'un métal physiologiquement acceptable à condition que $R^1$ représente un atome d'hydrogène lorsque $p=0$, et $R^2$ représente

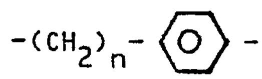

avec n allant de 1 à 4, ou un radical alkylène, arylène ou alkylènearylène substitué ou non.

A titre d'exemples de radicaux alkylène susceptibles d'être utilisés, on peut citer les radicaux éthylène, triéthylène, tétraméthylène, etc... On peut aussi utiliser des radicaux dérivant des hydrocarbures saturés ramifiés par enlèvement d'un atome d'hydrogène sur deux atomes de carbone différents de la chaîne de l'hydrocarbure.

A titre d'exemple de radicaux arylène susceptibles d'être utilisés, on peut citer le radical phénylène.

A titre d'exemple de polysaccharides modifiés présentant une activité anti-inflammatoire, on peut citer des dextranes modifiés par les groupes $-CH_2-COOR^1$ et les dextranes modifiés par des groupes de formule $-CH_2-COOR^1$,

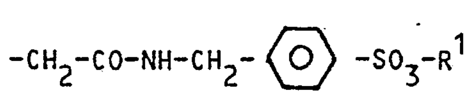

et

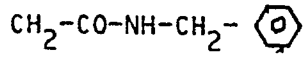 ,

ainsi que les celluloses modifiées par des groupes $-CH_2COOR^1$.

Lorsque le polymère est du polystyrène, les groupes fixés sur la chaîne de ce polymère pour lui conférer des propriétés anti-inflammatoires, sont généralement choisis parmi les groupes répondant aux formules $-SO_3R^1$, $-CH_2-CO-NH-X$,

$COY-COOR^1$, 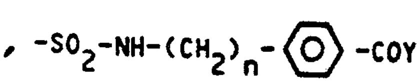 et 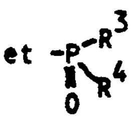

A titre d'exemples de tels objets, on peut citer ceux dans lesquels le polymère est du polystyrène comportant des groupes -COONa et ceux réalisés au moins en partie en polystyrène comportant des groupes $-SO_3Na$.

Dans ces formules, les radicaux $R^1$, $R^2$, X et Y peuvent représenter les mêmes radicaux que ceux utilisés pour la modification des polysaccharides. Les radicaux $R^3$ et $R^4$ représentent comme précédemment OH ou Y, et n est un entier de 1 à 4.

Les polymères modifiés de l'invention peuvent être préparés par des procédés classiques.

Ainsi, dans le cas où l'on désire obtenir des produits dans lesquels les groupes inhibiteurs comporte des groupes $-SO_3R^1$ et/ou $SO_2-Y$, on réalise généralement dans une première étape une chlorosulfonation du polymère par réaction avec de l'acide chlorosulfonique dans un solvant approprié, puis on transforme le groupe $SO_2Cl$ en groupe $-SO_3R^1$ en milieu basique et éventuellement en groupes $-SO_2-Y$ par réaction avec une quantité appropriée d'un acide aminé en milieu basique. La sulfonation du polymère a généralement lieu dans un mélange contenant du dichlorométhane et du nitrométhane et la réaction avec l'acide aminé dans un milieu contenant un mélange eau-dioxane.

Les groupes $-SO_2Y$ peuvent aussi être obtenus par transformation du groupe $-SO_2Cl$ par réaction d'un ester d'acide aminé en milieu basique en l'absence d'eau, généralement du dichlorométhane et de la triéthylamine. Dans ce cas, le polymère obtenu est saponifié par traitement à la soude concentrée suivi des lavages décrits ci-après.

Après fixation, on soumet le polymère à un lavage à l'eau suivi de lavages par des solutions de chlorure de sodium et de citrate de sodium, d'un équilibrage à un pH de 7,3 environ par plusieurs lavages avec du tampon de Michaelis, d'un nouveau lavage à l'eau et enfin d'un séchage. Ceci permet d'éliminer le plus complètement possible toute impureté susceptible d'interagir avec les constituants du plasma sanguin.

Dans le cas où l'on désire obtenir des polymères comportant des groupements $-CH_2CO-NH-X$, on prépare au cours d'une première étape les dérivés carboxyméthylés des polymères, puis on fixe au cours d'une deuxième étape les amines appropriées ou le chlorure de benzyle. Généralement, le couplage des amines sur les dérivés carboxyméthylés des polymères s'effectue à l'aide de N-éthoxycarbonyl-2-éthoxy-1,2-dihydroquinoléine (EEDQ).

Dans le cas où l'on fixe des groupements $-CH_2CO-Y$, on prépare au cours d'une première étape les dérivés carboxyméthylés des polymères, puis on fixe au cours d'une deuxième étape les aminoacides appropriés. Généralement le couplage des aminoacides sur les dérivés carboxyméthylés des polymères s'effectue à l'aide de N-éthoxycarbonyl-2-éthoxy-1,2-di-hydroquinoléine (EEDQ).

Dans le cas où l'on fixe des groupements $-COOR^1$, on fait réagir le polymère de départ avec du chlorure d'acétyle en présence de chlorure d'aluminium, puis les groupes acétyle sont transformés en carboxyle par oxydation à l'hypobromite de sodium.

Dans le cas où l'on fixe des groupements

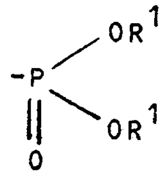 ,

on fait réagir le polymère de départ avec du trichlorure de phosphore en présence de chlorure d'aluminium. On dépare ensuite le produit par filtration, et on le fait réagir avec une solution aqueuse d'acide nitrique.

Dans le cas om l'on fixe des groupements

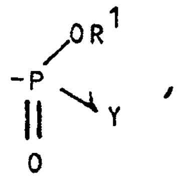

on fait réagir le polymère de départ avec du trichlorure de phosphore en présence de trichlorure d'aliminium, puis, après séparation (par exemple filtration) les groupes dichlorure de phosphoryle sont oxydés par le chlore pour obtenir les groupes tétrachlorure de phosphoryle. Les groupes ainsi obtenus peuvent réagir avec les acides aminés (ou les esters d'acides aminés) déjà cités.

Dans le cas, où l'on fixe des groupes

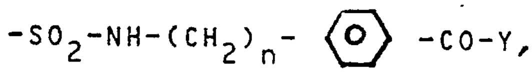

on réalise généralement dans une première étape une chlorosulfonation du polymère par réaction avec de l'acide chlorosulfonique dans un solvant approprié, puis on transforme les groupes $-SO_2Cl$ en groupes $-SO_3H$, puis on fixe sur le polymère sulfoné un acide de formule

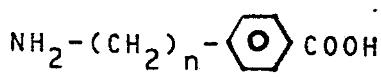

et on transforme ensuite les groupes COOH en groupes COY par réaction avec une quantité appropriée de l'acide aminé correspondant.

En raison des procédés utilisés pour la modification des polymères, ceux-ci comprennent généralement plusieurs groupes différents appartenant aux catégories précitées. Ainsi, lors de la fixation de groupes

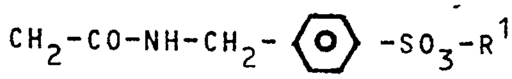

sur des polysaccharides, on obtient généralement des polysaccharides comportant, non seulement les groupes précités, mais également des groupes $-CH_2-COOR^1$ et des groupes

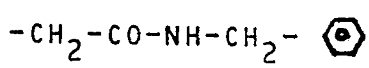 .

Dans ce cas, pour obtenir une bonne activité anti-inflammatoire du polymère, il est préférable que le nombre de groupes $-CH_2-COOR^1$ représente au moins 35% des groupes substituables du polymère de base et que le nombre de groupes

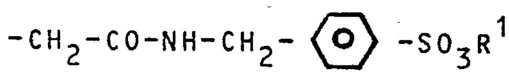

représente au moins 5% des groupes substituables du polymère de base.

Dans le cas des polystyrènes, lorsque l'on veut fixer sur le polymère des groupes de formule $-SO_2Y$, on fixe généralement également des groupes de formule $-SO_3R^1$.

Dans le cas des polystyrènes portant des groupes $-COOR^1$ ou

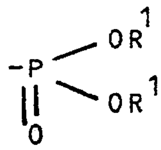 ,

on peut également fixer surles cycles benzéniques non substitués des groupes $-SO_3R^1$.

Selon l'invention, les objets à usage médical et/ou chirurgical réalisés en de tels polymères peuvent être en particulier des membranes de dialyse rénale ou de plasmaphérèse, des sondes et des supports pour épuration plasmatique.

Dans le cas des membranes de dialyse rénale ou de plasmaphérèse, on réalise ces membranes en polysaccharide, par exemple en cellulose, et l'on modifie en surface la membrane par fixation de groupes inhibiteurs choisis avantageusement parmi les groupes de formule $-CH_2-COOR^1$, $-CH_2-CO-Y$ et $-CH_2-CO-NH-X$ dans lesquels $R^1$, Y et X ont la signification donnée ci-dessus. Ces membranes peuvent être sous la forme de films ou de tubes.

Dans le cas de sondes à usage médical ou chirurgical, ces dernières peuvent être réalisées en un polymère ou un copolymère tel que ceux habituellement utilisés, par exemple en polyoléfine ou copolymère d'oléfine comme le polyéthylène et les copolymères d'éthylène et d'acétate de vinyle, mais selon l'invention leur surface qui est destinée à venir en contact avec l'organisme, est modifiée par greffage de polystyrène sur le polymère de base, puis fixation de groupes inhibiteurs de l'activation du système du complément de formule $-SO_2Y$, $-SO_3R^1$, $-CH_2CO-NH-X$, $-COOR^1$, $-CH_2-CO-NHR^2-SO_2Y$, COY,

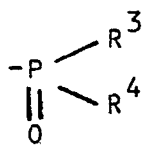 et/ou 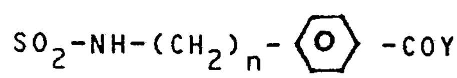

dans lesquelles $R^1$, $R^3$, $R^4$, X et Y ont la signification donnée ci-dessus.

La modification de surface peut être réalisée en particulier au niveau des passages transcutanés afin d'éviter les réactions inflammatoires de l'organisme.

Dans le cas de supports pour chromatographie d'affinité ou pour épuration plasmatique, on peut utiliser un support classique et modifier la surface de ce support en le revêtant par un polymère comportant dans sa chaîne des groupes substituables sur lesquels sont fixés de façon statistique les groupes inhibiteurs de l'activation du système du complément mentionnés ci-dessus.

Le support peut être constitué de grains de silice recouverts de dextrane réticulé comportant comme groupes inhibiteurs de l' activation du système du complément des groupes de formule $-CH_2COOR^1$, $-CH_2-CO-Y$ et/ou $-CH_2-CO-NH-X$.

L'invention a encore pour objet l'utilisation de polymères ou copolymères comportant dans leur chaîne des groupes substituables sur lesquels sont fixés de façon statistique des groupes inhibiteurs de l'activation du système du complément répondant à la formule :

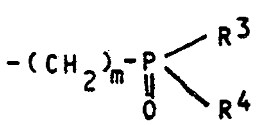

dans laquelle $R^3$ et $R^4$ qui peuvent être identiques ou différents, représentent $OR^1$ ou Y avec $R^1$ représentant un atome d'hydrogène ou d'un métal physiologiquement acceptable et Y représentant un radical dérivé d"n acide aminé lié au pont P=0 par sa fonction amine, et m est égal à 0 ou est un nombre entier allant de 1 à 15, pour la fabrication d'objets à usage médical ou chirurgical destinés à traiter ou réduire l'inflammation.

A titre d'exemple, dans ce cas, $R^3$ peut représenter ONa, $R^4$ représenter OH, et m être égal à 0.

D'autres caractéristiques et avantages de l'invention apparaîtront mieux à la lecture des exemples suivants donnés bien entendu à titre illustratif et non limitatif, en référence au dessin annexé sur lequel :

- la figure 1 est un diagramme illustrant l'activation du système du complément en fonction du taux de groupes $-CH_2COONa$ du Séphadex carboxyméthylé,

- la figure 2 est un diagramme illustrant l'activation du système du complément par le Séphadex carboxyméthylé en fonction du taux de groupes -$CH_2$-COONa,
- la figure 3 est un diagramme illustrant l'effet inhibiteur du Séphadex carboxyméthylé en fonction de la quantité de polymère utilisée,
- la figure 4 est une courbe d'étalonnage pour évaluer l'ef+et inhibiteur des polymères de l'invention,
- la figure 5 est un diagramme représentant l'effet inhibiteur du Séphadex carboxyméthylé en fonction de la quantité de polymère utilisée,
- la figure 6 est un diagramme illustrant l'effet inhibiteur du CMBS Séphadex en fonction de la quantité de polymère utilisée,
- la figure 7 est un diagramme illustrant l'effet inhibiteur des polystyrènes substitués en fonction des quantités de polymère utilisées, et

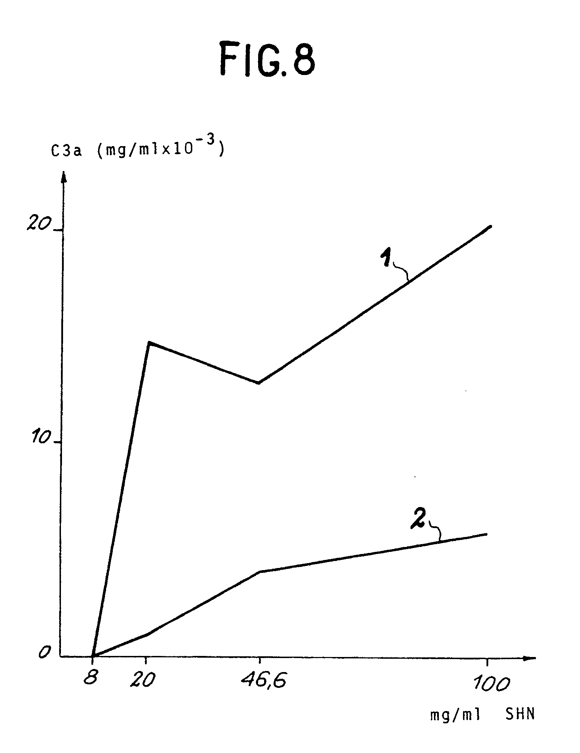

- la figure 8 est un diagramme illustrant l'effet d'activation du système du complément par la cellulose en fonction de la quantité de cellulose utilisée.

EXEMPLE 1 : Polydextrane comportant des groupes $CH_2COONa$ de formule :

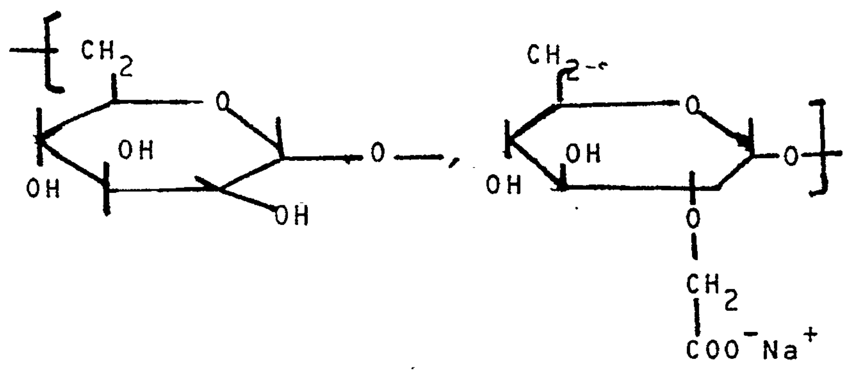

a) -Préparation

On utilise comme produit de départ le polydextrane réticulé, commercialisé sous la marque Séphadex G 25, qui est un dextrane réticulé par l'épichlorhydrine, et on le modifie par carboxyméthylation en utilisant la méthode décrite par BOUTTEMY dans le cas de la cellulose.

On vérifie ensuite les propriétés des polydextranes réticulés ainsi modifiés (CM Séphadex) présentant des taux de carboxyméthylation allant de 18 à 95,5%, c'est-à-dire ayant un taux de groupes -$CH_2COOR^1$ qui représente de 18% à 85,5% des groupes substituables du polydextrane de départ.

b) - Effet du polymère sur l'activation du système du complément

Tout d'abord, on broie le CM Séphadex contenant des motifs $CH_2COONa$ afin d'obtenir un diamètre moyen de particules de 15 $\mu$m, puis on élimine les fines particules et on conditionne la poudre dans un tampon physiologique à pH 7,4 (tampon $VBS^{++}$ ou tampon MgEGTA). Le taux de gonflement du polymère est voisin de 4,65 ± 0,05 (en volume).

Le tampon $VBS^{++}$ est préparé à partir d'une solution cinq fois plus concentrée, conservée au froid, comprenant pour 42,5 $g.l^{-1}$ de NaCl, 1,875 $g.l^{-1}$ de Na 5,5-diéthylbarbital et 2,875 $g.l^{-1}$ de Na 5,5-diéthylbarbiturique et ayant un pH de 7,4.

A 100 ml de cette solution diluée ($VBS^{-}$), on ajoute 0,5 ml de $CaCl_2$ $3.10^{-2}$ molaire et 0,5 ml de $MgCl_2$ 0,1 molaire : on obtient ainsi le tampon $VBS^{++}$.

Le tampon MgEGTA est réalisé à partir d'une solution-mère conservée à 2° C comprenant 6,08 g de EGTA (0,16 M), 0,816 g de $MgCl_2$ (0,04 M) et 75 ml d'eau distillée en ajustant à un pH de 7,4-7,5 par de la soude 10N et en ajustant ensuite à 100 ml par addition d'eau distillée. Lors des manipulations, on dilue le tampon au 1/20° en $GVB^{-}$ de la solution-mère afin d'obtenir un tampon MgEGTA contenant 8 mmoles de EGTA et 2 mmoles de $Mg^{2+}$.

On réalise ensuite une série d'essais ayant pour but de vérifier l'effet du CM Séphadex sur l'activation du système du complément par la voie classique ou par la voie alterne.

Dans les deux cas, on utilise 8,75 mg de CM Séphadex par millilitre de sérum humain normal dilué au 1/4 dans le tampon correspondant.

Dans un tube à hémolyse, on introduit 26,2 mg de CM Séphadex dans 1,5 ml du tampon VBS ou MgEGTA et 1,5 ml de sérum humain normal dilué au 1/2 dans le même tampon MgEGTA ou $VBF^{++}$. On laisse incuber 1 heure à 37° sous agitation et on mesure l'activation de la voie classique et alterne par le dosage du CH50 et l'activation de la voie alterne par le dosage de la protéine B.

1. Dosage de l'activation par la somme des voies classiques et alternes

Dans ce cas, l'activation est mesurée en évaluant la capacité du sérum à lyser des globules rouges de mouton (GRM) recouverts d'anticorps anti-GRM. Ces cellules (EA) sont lavées deux fois en tampon $VBS^{2+}$ et ajustées à $1,5.10^8$/ml, ce qui correspond à une densité optique de 0,429 à 412 nm pour une suspension cellulaire diluée au 1/30ème dans l'eau distillée. Le dosage est effectué de la façon suivante.

Dans un tube à hémolyse, on introduit 0,2 ml de sérum humain normal (SHN) à doser dilué au 1/20ème en $VBS^{2+}$ (100 ml de tampon $VBS^{-}$ + 0,5 ml de $CaCl_2$ 0,03 M et 0,5 ml de $MgCl_2$ 0,1 M), puis on fait des dilutions successives dans du sérum humain normal de 2 en 2 selon le protocole suivant : et on ajoute 0,2 ml des érythrocytes de mouton recouverts d'anticorps (EA) à $1,5.10^8$/ml).

| N° tubes | 1 | 2 | 3 | 4 | 5 | 6 | 7 |
|---|---|---|---|---|---|---|---|
| SHN 1/20ème en $VBS^{++}$ (ml) | 0,2 | 0,2 | | | | | |
| $VBS^{++}$ (ml) | | 0,2 | 0,2 | 0,2 | 0,2 | 0,2 | 0,2 |
| EA (ml) | 0,2 | 0,2 | 0,2 | 0,2 | 0,2 | 0,2 | 0,2 |

Pour mesurer la lyse spontanée : $T_0$ des cellules, on introduit dans un tube à hémolyse :

- 0,2 ml de tampon $VBS^{2+}$
- 0,2 ml d'érythrocytes de mouton recouverts d'anti-corps.

Pour mesurer la lyse totale des cellules : $T_{100}$, on introduit dans un tube à hémolyse : 0,2 ml de tampon $VBS^{2+}$,0,2 ml d'érythrocytes de mouton EA.

On porte les différents tubes à hémolyse à 37° C et on les maintient à cette température pendant 40 min sous agitation. On arrête ensuite la réaction avec 2,4 ml de NaCl froid 0,15N, sauf pour le tube de mesure de la lyse totale où l'on introduit 2,4 ml d'eau distillée. On centrifuge alors les tubes à environ 2800 tours/min avant de lire la densité optique DO du surnageant à 412 nm.

Le pourcentage de lyse y qui correspond à :

$$y = \frac{DO - T_0}{T_{100} - T_0}$$

est mesuré et on calcule le pourcentage de protéines du système de complément résiduel à partir du degré de lyse des érythrocytes de mouton (EA) par référence au témoin sérum qui n'a pas été mis en contact avec le CM Séphadex.

Plus y est élevé (DO élevé), moins le système du complément a été activé par le polymère. Les valeurs peuvent être exprimées en unité par ml, l'unité hémolytique 50 ($CH_{50}$) qui définit la quantité de sérum nécessaire à la lyse de 50% des EA. Le $CH_{50}$ est calculé de la façon suivante : à partir du degré de lyse y, on rapporte sur un graphique la quantité de sérum dilué (0,2 ml ; 0,1 ml, etc...) en fonction de

$$\frac{y}{1-y}$$

et on lit la quantité de sérum nécessaire pour obtenir 50% de lyse des EA

$$\left(\frac{y}{1-y}=1\right).$$

La précision de ce dosage est évaluée à 10%.

Les résultats obtenus avec les CM Séphadex ayant des taux de groupes $-CH_2COONa$ allant de 18 à 95,5% sont donnés sur la figure 1 qui représente le $CH_{50}$ résiduel en fonction de la teneur en groupes $-CH_2COONa$ (en %) du polymère. Sur cette figure, on voit que l'activation du système du complément diminue avec la teneur en groupes $-CH_2COONa$ du polymère et que pour 95,5% de groupes $-CH_2COONa$, on n'obtient pratiquement aucune activation du système du complément.

Sur cette figure, la ligne en tirets se rapporte au tube témoin sans polymère.

2. Activation par voie alterne

Le dosage de l'activation de la voie alterne se fait par un dosage hémolytique de la protéine B en utilisant des érythrocytes de mouton recouverts d'anti-corps et de fragments C3b. Ces cellules (EAC4-3b) sont suspendues dans un tampon physiologique à pH 7,4 $DGVB^{++}$ obtenu en ajoutant à 100 ml de tampon $VBS^{--}$ 1 ml de gélatine (à 10g pour 100 ml d'eau), 100 ml de dextrose (25g $\alpha$ $D^{+}$ glucose pour 100 ml d'eau), 1 ml de $CaCl_2$ 0,03M et 1 ml de $MgCl_2$ 0,1M. Le dextrose ajouté favorise la formation de la C3 convertase car le milieu est rendu hypotonique. La gélatine diminue l'absorption non spécifique des protéines.

Pour le dosage hémolytique, les cellules sont ajustées à $1.10^{8}$/ml (densité optique de 0,286 nm pour une suspension cellulaire diluée au 1/30 dans l'eau distillée). On ajoute en excès à cette suspension cellulaire les protéines nécessaires à la formation de la C3 convertase alterne sauf la protéine B dosée dans le sérum. Ainsi, on ajoute le facteur $\overline{D}$ et le facteur P dilués au 1/100 à la suspension cellulaire lavée préalablement deux fois en tampon $DGVB^{++}$.

Le dosage hémolytique se fait de la façon suivante :

Dans un tube à hémolyse, on introduit :

- 0,1 ml de sérum humain normal à doser dilué au 1/40000 en tampon $DGVB^{++}$,
- 0,1 ml de EAC4,3b ; 0,1 ml de P/100 et 0,1 ml de D/100.

On fait différentes dilutions successives de 2 en 2 du sérum à doser selon le protocole suivant :

| N° tubes | 1 | 2 | 3 | 4 | 5 | 6 | 7 |
|---|---|---|---|---|---|---|---|
| SHN 1/40000 en $DGVB^{++}$ (ml) | 0,1 | 0,1 | | | | | |
| $DGVB^{++}$ (ml) | | 0,1 | 0,1 | 0,1 | 0,1 | 0,1 | 0,1 |
| EAC4 C3b, P/100-D/100 (ml) | 0,1 | 0,1 | 0,1 | 0,1 | 0,1 | 0,1 | 0,1 |

Pour mesurer la lyse spontanée TO et la lyse totale des cellules $T_{100}$, on introduit dans un tube à hémolyse :

- 0,1 ml de EAC4C3b, P/100, D/100 et
- 0,1 ml de tampon $DGVB^{++}$.

On porte les tubes au bain-marie à 30°C sous agitation pendant 30 min de façon à former la C3 convertase amplificatrice alterne : C3bBbP.

On ajoute ensuite 0,3 ml de sérum de rat dilué au 1/20 dans un tampon physiologique contenant de l'acide éthylénediaminetétracétique 40mM (EDTA) pour apporter les protéines nécessaires à la lyse en

excès à partir du $C_5$...$C_9$ sans faire intervenir les autres composants du sérum qui seront bloqués par l'EDTA. On incube au bain-marie à 37° C sous agitation pendant 60 min, puis on arrête la réaction avec 1,6 ml de NaCl froid 0,15M, sauf pour le tube de lyse totale où on ajoute 1,6 ml d'eau distillée. On centrifuge à 2800 t/min avant de mesurer la densité optique (DO) du surnageant à 412 nm.

La densité optique DO permet de mesurer le pourcentage de lyse :

$$Y = \frac{DO - T_0}{T_{100} - T_0}$$

Plus y est élevé, moins le polymère a activé la voie alterne du complément.

On calcule le pourcentage de protéine B résiduel du sérum humain normal préalablement incubé avec le CM Séphadex à partir du pourcentage de lyse érythrocytaire par référence au témoin sérum n'ayant pas été mis en contact avec le CM Séphadex. On peut ainsi définir la valeur Z=-lr(1-y) correspondant au nombre de sites convertasiques par cellule.

Les résultats obtenus sont donnés sur la figure 2 qui représente le pourcentage de protéine B résiduel en fonction du taux de substitution en groupes $-CH_2COONa$ du Séphadex. Comme précédemment, on constate que l'inactivation du système du complément est directement proportionnelle au taux de groupes $-CH_2COONa$. Sur cette figure, la ligne en tirets correspond à l'essai effectué avec le sérum humain normal en l'absence de CM Séphadex.

3. Dosage radioimmunologique des deux voies du complément

Une gamme d'étalonnage est construite selon le protocole décrit ci-après : une volume de $^{125}$I-C3a (C3a*) est ajouté à un volume de C3a froid en quantité variable connue. Un anticorps anti C3a (AC1) est ajouté, incubé 30 minutes à l'ambiante, puis un deuxième anticorps antiAC1 est ajouté (AC2) qui se lie au complexe déjà formé. La réaction est arrêtée avec du NaCl 0,15M froid, le tout est centrifugé et on compte la radioactivité du précipité formé. Ceci permet de construire le graphe AC2-AC1-C3a* en fonction de la quantité de C3a froid. Les sérums à doser sont traités par un agent précipitant (Polyéthylène glycol) des composés de haut poids moléculaire. Après centrifugation, un volume du surnageant à doser est ajouté à un volume de C3a* selon la technique décrite dans l'étalonnage. Selon le milieu dans lequel le sérum a été placé en présence du polymère, on mesure par cette méthode soit l'activation des deux voies (en $VBS^{++}$) soit l'activation de la voie alterne (en Mg EGTA).

c) - Effet inhibiteur du polymère sur l'activation du système du complément

1. Activation par le Séphadex G 25

On étudie l'effet du Séphadex carboxyméthylé sur l'activation du système du complément par le Séphadex G25 qui est connu pour activer le système du complément. Le Séphadex G25 qui constitue la résine activatrice est conditionné dans le tampon $VBS^{++}$ et présente une granulométrie d'environ 15 µm. On met en contact le Séphadex G25 à concentration constante (8,75 mg/ml de sérum humain normal) et le Séphadex G25 carboxyméthylé à raison de 5 mg, 10 mg ou 15 mg/ml de sérum humain normal dilué au 1/4. Les témoins sont le sérum humain normal dilué au 1/4 en $VBS^{++}$,

- le Séphadex G25 à raison de 8,75 mg/ml en sérum humain normal dilué au 1/4 en $VBS^{++}$,
- le Séphadex G25 carboxyméthylé à 95% à raison de 5, 10, 15 mg/ml de sérum humain normal dilué au 1/4 en tampon $VBS^{++}$.

Les tubes sont incubés pendant 1 heure à 37° C sous agitation et on détermine le $CH_{50}$ pour mesurer l'activation des deux voies du système du complément.

Les résultats obtenus sont donnés sur la figure 3 où la droite 1 représente le taux de $CH_{50}$ résiduel en fonction de la teneur en Séphadex carboxyméthylé exprimé en mg/ml de sérum humain normal. Sur cette figure, la courbe 2 illustre les résultats obtenus avec le CM Séphadex à 95% seul et la droite 3 se rapporte au sérum humain normal seul.

Sur cette figure, on voit que le taux d'inhibition de l'activation du système du complément augmente avec la quantité de Séphadex G25 carboxyméthylé utilisé dans l'essai. Ainsi, 10 mg/ml SHN 1/4 de Séphadex carboxyméthylé à 95% inhibe 50% de l'activation globale du système du complément par 8,75 mg de Séphadex par ml de SHN 1/4 en $VBS^{++}$.

2. Activation par les globules rouges de lapin

Dans cet essai, on étudie l'effet du CM Séphadex sur l'activation du système du complément par les globules rouges de lapin en mesurant l'activation par un dosage hémolytique.

Pour ce dosage, on construit une courbe d'étalonnage afin d'établir la concentration de sérum humain normal (SHN) capable d'hémolyser 50 ou 100% de globules rouges de lapin (GRL) de la façon suivante. (Différentes doses 10, 11,...,36 µl) de SHN sont ajustées à 250 µl en tampon MgEGTA auxquels on ajoute 100 µl de GRL à $10^8$/ml. On laisse incuber à 37° C pendant 40 min au bain-marie sous agitation, puis on centrifuge à 3000 t/min et on mesure la densité optique DO du surnageant à 412 nm. On détermine le degré de lyse des GRL en fonction de la quantité de SHN présente dans le tube au moment du test. Les résultats obtenus sont donnés sur la figure 4 qui représente la courbe d'étalonnage obtenue dans ces conditions, soit le pourcentage de lyse des GRL en fonction de la quantité de sérum humain normal utilisée.

Pour évaluer l'effet du Séphadex G25 carboxyméthylé, on ajoute différentes doses de ce polymère ajustées à 250 µl avec le tampon MgEGTA à une dose constante de SHN capable d'hémolyser 50 ou 100% des GRL. On ajoute 100 µl de GRL $10^8$ hématies/ml en MgEGTA, on laisse incuber à 37° C au bain-marie pendant 40 min sous agitation, puis on centrifuge à 3000 t/min et on mesure la densité optique du surnageant à 412 nm.

Le pourcentage de lyse des GRL est déterminé en fonction de la quantité de polymère ajoutée. Un blanc sans sérum humain normal a été fait avec le Séphadex G25 carboxyméthylé afin de vérifier que le fait de mettre en contact ce polymère avec les GRL ne provoquait pas la lyse de ces derniers. La densité optique mesurée dans ces conditions ne montre pas un tel effet.

Les résultats obtenus sont donnés sur la figure 5 où la courbe représente le pourcentage de lyse des GRL de lapin pour différents polymères. Sur cette figure, on voit que l'addition de 3 à 5 mg de Séphadex carboxyméthylé à 95% conduit à un pourcentage de lyse très faible des GRL et que ce polymère a donc un effet inhibiteur sur l'activation du système du complément.

EXEMPLE 2 : Polydextrane réticulé, modifié répondant à la formule suivante :

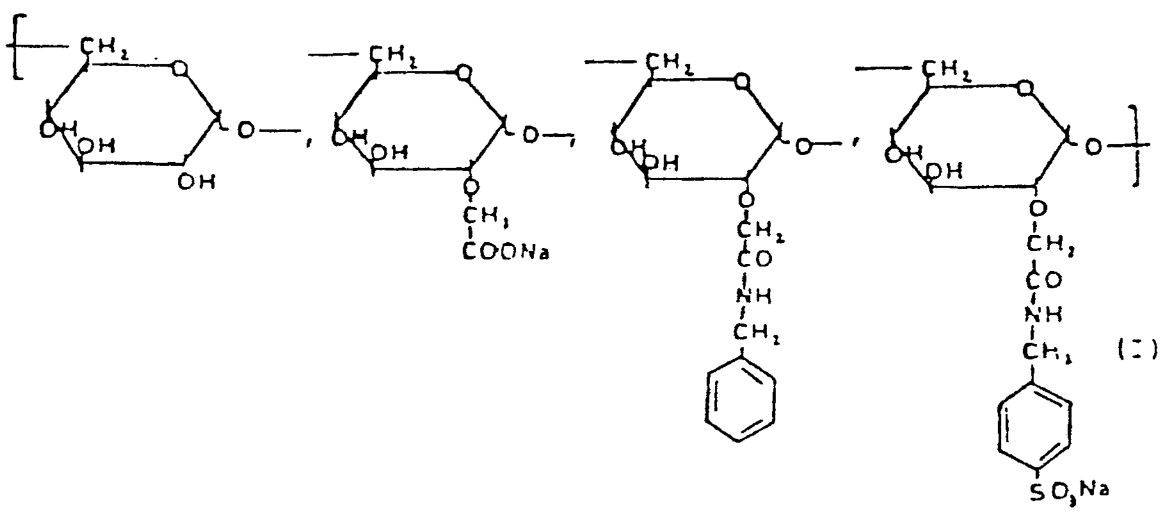

(I)

a) - Préparation du polymère

Ce produit est préparé de la façon suivante. On part de Séphadex G25 carboxyméthylé à 76% mis sous forme acide et on fixe sur celui-ci de la benzylamine en opérant de la façon suivante.

Dans un ballon de 250 $cm^3$, on disperse sous agitation 5g de carboxyméthyl-Séphadex dans 60 $cm^3$ d'eau ; on ajoute lentement une solution de 20 g de N-éthoxycarbonyl-2-éthoxy-1,2 dihydroquinoléine (EEDQ) dissous dans 160 ml d'éthanol absolu et on laisse agir une demi-heure. On ajoute alors 4,4 $cm^3$ de benzylamine et on laisse le mélange sous agitation pendant 24 h, puis on filtre pour récupérer le produit. On lave abondamment à l'eau distillée puis à l'éthanol absolu et encore une fois à l'eau, puis on sèche à l'étuve sous vide à 40° C pendant 24 heures.

On soumet ensuite le produit ainsi obtenu à une sulfonation en opérant de la façon suivante.

5g du produit obtenu précédemment sont dispersés dans 500 $cm^3$ de nitrométhane sous agitation. On

ajoute alors lentement 2,8 $cm^3$ d'acide chlorosulfonique et on laisse agir pendant 2 heures. On récupère le produit par filtration, on le lave abondamment au nitrométhane puis à l'eau distillée et on met le produit obtenu dans une solution de soude 1N pendant 2 heures afin de l'hydrolyser totalement. On récupère le produit par filtration, on le lave abondamment à l'eau distillée et on le sèche à l'étuve sous vide à 40° C pendant 24 h.

Le polymère ainsi obtenu comprend :

- 53% de groupes $-CH_2COONa$,
- 10% de groupes $-CH_2CONHCH_2-$ 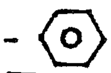 $-SO_3Na$,
- 13% de groupes $CH_2CONHCH_2$ 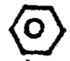 ,
- 24% de groupes OH non substitués.

Le polymère (CMBS) ainsi obtenu a un diamètre moyen de particules de 30 µm. Après élimination des fines particules, on le conditionne dans un tampon physiologique à pH 7,4 ($VBS^{-}$). Le taux de gonflement est voisin de 4,65 ± 0,05 en volume.

b) - Effet du polymère sur l'activation du système du complément.

On opère comme dans l'exemple 1 pour vérifier l'effet du polymère sur l'activation du système du complément par les deux voies ou par la voie alterne seule. Les essais sont réalisés dans les mêmes conditions que celles de l'exemple 1. Dans ces conditions, on n'obtient pas d'activation du système du complément aussi bien par la voie classique que par la voie alterne.

c) - Effet du polymère sur l'activation du système du complément par les globules rouges de lapin.

On vérifie cet effet dans les mêmes conditions que celles de l'exemple 1. Les résultats obtenus sont donnés sur la figure 6 qui représente le pourcentage de lyse des GRL en fonction de la teneur en polymère (mg)

Sur cette figure, on voit que ce polymère est légèrement moins inhibiteur que le Séphadex carboxyméthylé de l'exemple 1, puisqu'il faut plus de CMBS que de CM Séphadex pour obtenir le même résultat. Néanmoins, le CMBS a un effet inhibiteur sur l'activation du système du complément par les globules rouges de lapin.

EXEMPLE 3 : Polystyrène modifié par des groupes $-SO_3Na$ répondant à la formule :

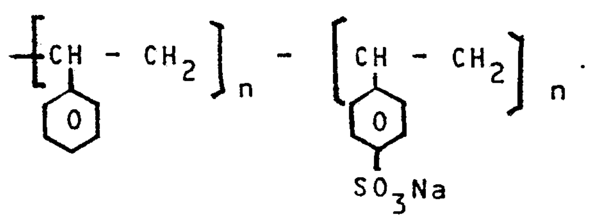

a) - Préparation du polymère

On laisse gonfler une nuit à la température ambiante 25 g de polystyrène dans 360 ml de dichlorométhane. On ajoute alors 140 ml d'acide chlorosulfonique, puis on agite la suspension pendant 7 h à température ambiante. On filtre alors la résine brute, on la lave avec précautions au moyen de dichlorométhane et d'acétone, puis on la sèche sous vide à 50° C. On hydrolyse ensuite le polystyrène sulfoné ainsi obtenu par la soude 2N à la température ambiante, puis on filtre, on lave à l'eau et on sèche sous vide. Le produit obtenu ($PS-SO_3Na$) est substitué à 68% par $-SO_3Na$, son taux de gonflement est de 1,93 et le diamètre moyen de particules est de 30 µm.

b) - Effet sur l'activation du système du complément par les globules rouges de lapin

On détermine cet effet dans les mêmes conditions que celles des exemples 1 et 2.

Les résultats obtenus sont donnés sur la figure 7 où la courbe 1 représente le pourcentage de lyse des globules rouges de lapin en fonction du poids de polymère utilisé (en mg).

Sur cette figure, on voit que le polystyrène comportant des groupes $SO_3Na$ a un effet inhibiteur sur l'activation du système du complément par les globules rouges de lapin puisque 0,9 mg inhibe 50% des GRL.

EXEMPLE 4 : Polystyrène comportant des groupements $-SO_3Na$ et $-SO_2Y$ avec Y représentant un radical dérivé de l'acide aspartique répondant à la formule :

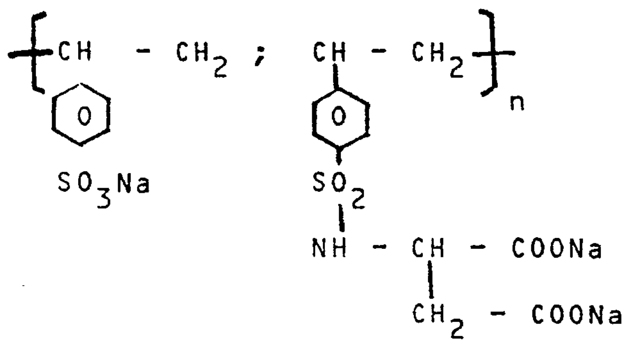

a) - Préparation du polymère

On soumet tout d'abord, comme dans l'exemple 3, du polystyrène à une chlorosulfonation, puis on fixe sur ce polystyrène chlorosulfoné de l'acide aspartique en opérant de la façon suivante.

On dissout 15 mmol de chlorhydrate d'ester méthylique d'acide aspartique dans 150 ml de dichlorométhane auquel on a ajouté 1,5 g de triéthylamine. On ajoute alors 2 g du polystyrène chlorosulfoné, puis, au bout de 2h, on ajoute 1g de triéthylamine.

On arrête la réaction après une nuit. On filtre alors le polymère, on le lave abondamment à l'alcool, puis avec de la soude 2M, 1M, $10^{-2}$M et de l'eau et on le sèche sous vide. On obtient ainsi un polymère ayant la formule ci-dessus dans laquelle le nombre de groupes $-SO_2Y$ représente 92% des motifs substitués.

Ce polymère (PS-$SO_2$AA) a un taux de gonflement de 1,89 et un diamètre moyen de particules de 14,3 µm.

b) - Effet du polymère sur l'activation du système du complément par les globules rouges de lapin

On détermine cet effet dans les mêmes conditions que celles de l'exemple 1 après avoir conditionné les résines dans le tampon MgEGTA.

Les résultats obtenus sont donnés sur la figure 7 sur laquelle la courbe 2 se rapporte à ce polymère (PS-$SO_2$AA). On voit que ce polymère a un effet inhibiteur plus prononcé que le polystyrène sulfoné (PS-$SO_3$Na) de l'exemple 3, puisque 0,3 mg de PS-$SO_2$AA suffit inhiber 50% des GRL, alors que 0,9 mg de PS-$SO_3$Na sont nécessaires pour obtenir le même résultat.

EXEMPLE 5 : Polystyrène modifié par des groupes COONa de formule :

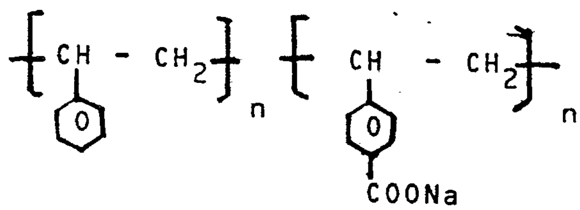

a) - Préparation du polymère

La résine polystyrène (12g) est mise à gonfler dans 150 ml de dichlorométhane pendant une nuit. Le ballon est ensuite plongé dans la glace et on ajoute 9,9 ml de $CH_3COCl$ puis 15g de $AlCl_3$ par petites quantités. Le mélange est agité pendant deux jours à température ambiante, puis il est filtré et lavé dans des mélanges dioxanne /eau/ acide chlorhydrique afin d'éliminer l'alumine formée puis à la soude et à l'eau, enfin séché. La résine précédente est alors placée dans un bain de glace dans 100 ml d'un mélange dioxanne /eau, on lui ajoute 48g de NaOH dans 200 ml d'eau puis doucement 16 ml de $Br_2$. Après deux jours à l'ambiante, la résine est lavée à la soude 1 M puis $10^{-2}$M, puis séchée. Le polymère obtenu est substitué à plus de 90%.

b) - Effet du polymère sur l'activation du système du complément

On vérifie cet effet dans les mêmes conditions que celles de l'exemple 1. Dans ces conditions, on a trouvé que 10 mg de polystyrène modifié par COONa n'ont aucun effet sur l'activation du système du complément.

c) - Effet du polymère sur l'activation du système du complément par le Séphadex G25

On vérifie cet effet dans les mêmes conditions que celles de l'exemple 1. Les résultats obtenus montrent que ce polymère a un effet inhibiteur sur l'activation du complément par le Séphadex G25.

EXEMPLE 6 : Polystyrène modifié par des groupements

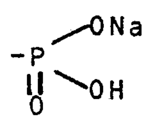

répondant à la formule :

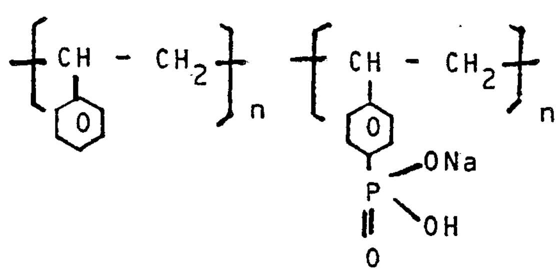

a) - Préparation du polymère

A 2g du polymère de départ, on ajoute 14 ml de $PCl_3$ puis, après 30 minutes à température ambiante,

1,86g de $AlCl_3$. Le mélange est alors chauffé à 80° C pendant 5h puis la résine résultante est filtrée, rincée au chloroforme, puis au dioxanne puis traitée par 300 ml d'un mélange dioxanne/acide nitrique /eau (2/1/1 en volume) pendant 48h. La résine obtenue est alors traitée après filtration par de la soude diluée, puis elle est séchée sous vide. Le polymère obtenu est substitué à environ 50%.

b) - Effet du polymère sur l'activation du système du complément

Cet effet est déterminé dans les mêmes conditions que celles de l'exemple 1, sur un polymère ayant 50% de groupes substituables occupés par :

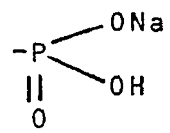

en utilisant 10 mg du polymère. On vérifie que dans ces conditions, le pourcentage de protéines B résiduel est de 100%, ce qui montre que ce polymère n'active pas le système du complément.

c) - Effet du polymère sur l'activation du système du complément par le Séphadex G25

On détermine cet effet dans les mêmes conditions que celles de l'exemple 1 en utilisant 10 mg de polymère et l'on constate que ces 10 mg suffisent à inhiber l'activation du système du complément par le Séphadex G25.

EXEMPLE 7 : Cellulose modifiée par des groupes $CH_2$-COONa.

a) - Préparation du polymère

On met 281 mg soit 1,73 mmotif de cellulose commercialisée sous la marque Cuprophane (Hospal Industrie, Meyzieu) 150 PT lot 296 598, dans 9,2 ml de NAOH 3M. On ajoute ensuite 0,49g (5,19 mmol) d'acide monochloracétique. Ceci correspond à un rapport de carboxyméthylation (acide/motif) de 3,5. On agite le mélange à 20° C pendant 17 heures. On sépare ensuite le polymère de la solution et on le lave dans du méthanol puis dans de l'eau. On ajuste le pH à 7 par addition d'acide acétique et on le lave à l'éthanol, puis on le filtre et on le sèche.

Un dosage acide-base des fonctions carboxyméthylées effectué par dosage automatique et par spectrométrie infrarouge montre que l'on obtient un taux de substitution d'environ 1 milliéquivalent par gramme de polymère sec.

b) - Effet du polymère sur l'activation du système du complément

On vérifie tout d'abord que le Cuprophane non modifié est activateur du système du complément en réalisant des essais par dosage radioimmunologique selon le mode opératoire de l'exemple 1 c)3 avec du sérum humain normal dilué au 1/4 et différentes quantités de Cuprophane non modifié dans le tampon $VBS^{++}$ et le tampon MgEGTA en réalisant l'incubation pendant une heure à 37° C. Avant de réaliser les essais, on lave le Cuprophane dans NaCl 0,15M et dans le tampon correspondant ($VBS^{++}$ ou MgEGTA).

Les résultats obtenus sont donnés sur la figure 7 qui représente la quantité de C3a du surnageant (en ng/ml.$10^{-3}$) en fonction de la quantité de polymère utilisé (en mg/ml de SHN). Sur cette figure, la courbe 1 se rapporte aux essais effectués avec le tampon $VBS^{++}$ et la courbe 2 aux essais réalisés avec le tampon MgEGTA. On constate ainsi que le Cuprophane active le système du complément par la voie classique et par la voie alterne.

En revanche, on précise dans les mêmes conditions, en utilisant le tampon $VBS^{++}$ le Cuprophane carboxyméthylé à une dose de 48 ng/ml de SHN n'active pas le système du complément.

De même, des essais réalisés selon le mode opératoire de l'exemple 1 b)1, ont montré que le Cuprophane carboxyméthylé n'activait pas le système du complément par les voies classique et alterne.

EXEMPLE 8 : réalisation d'une membrane de dialyse rénale en cellulose carboxyméthylé.

On part de membranes classiques de dialyse réalisées en cellulose, qui peuvent avoir la forme de plaques planes ou de fibres creuses, et on les soumet à une carboxyméthylation en suivant le mode opératoire décrit dans l'exemple 7 de façon à modifier la surface de la membrane qui sera en contact avec le sang humain, par fixation sur celle-ci de groupes $-CH_2COONa$.

On obtient ainsi des membranes de dialyse rénale présentant la propriété de ne plus activer le système du complément.

EXEMPLE 9 : Réalisation d'une sonde en polyéthylène

On part d'une sonde en polyéthylène et on greffe sur les parties de la sonde qui seront destinées à venir en contact avec l'organisme du polystyrène en exposant les parties de la sonde à modifier immergées dans une solution de styrène aux rayonnements $\gamma$ d'une source de cobalt 60, en l'absence d'oxygène. On modifie ensuite le polystyrène ainsi greffé par chlorosulfonation en le faisant gonfler dans un mélange de chlorométhane et de nitrométhane, puis en faisant passer de l'acide chlorosulfonique pendant 20 min en circulation et en renouvelant cette opération une fois. Après chlorosulfonation, on traite la sonde par un ester d'acide aminé en solution dans le même solvant pendant 24 h. On saponifie ensuite par NaOH, puis on lave à l'eau et au tampon de Michaelis pendant un temps suffisant.

On obtient ainsi une sonde capable d'inhiber l'activation du système du complément au niveau des passages transcutanés.

## Revendications

1. Objet insoluble à usage médical ou chirurgical, caractérisé en ce qu'il est réalisé au moins en partie en un polymère ou un copolymère comportant dans sa chaîne des groupes substituables sur lesquels sont fixés de façon statistique des groupes inhibiteurs de l'activation du système du complément choisis parmi les groupes répondant aux formules suivantes :

   1) - $-(CH_2)_mCOOR^1$ dans laquelle $R^1$ représente un atome d'hydrogène ou d'un métal physiologiquement acceptable et m est égal à 0 ou est un nombre entier de 1 à 15,

   2) - $-CH_2-CO-NH-X$ dans laquelle X représente $-R^2-H$ où $R^2$ représente

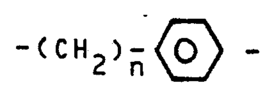

   avec n étant un nombre entier de 1 à 4, ou un radical alkylène, arylène ou alkylènearylène substitué ou non,

   3) - $-(CH_2)_m-CO-Y$, dans laquelle Y représente un radical dérivé d'un acide aminé ou d'un sel d'acide aminé lié au pont -CO- par sa fonction amine, et m est égal à 0 ou est un nombre entier de 1 à 15,

   4) - $-SO_3R^1$, dans laquelle $R^1$ a la signification donnée ci-dessus,

   5) -

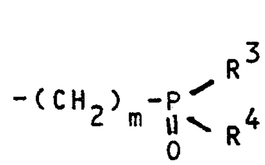

   dans laquelle $R^3$ et $R^4$ qui peuvent être identiques ou différents, représentent $OR^1$ ou Y avec $R^1$ ayant la signification donnée ci-dessus et Y représentant un radical dérivé d'un acide aminé lié au pont -PO- par sa fonction amine, et m est égal à 0 ou est un nombre entier allant de 1 à 15, à condition que $R^3$ et $R^4$ représentent OH ou Y lorsque m est égal à 0.

   6) -

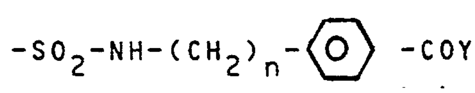

   dans laquelle Y représente un radical dérivé d'un acide aminé lié au pont CO par la fonction amine

et n est un nombre entier de 1 à 4, à condition que le polymère ou le copolymère ne comporte qu'un seul type de groupes inhibiteurs lorsque ces groupes ont pour formule $-SO_3R^1$ ou $-(CH_2)_mCOY$ avec m étant égal à 1.

2. Objet selon la revendication 1, caractérisé en ce que le polymère est un polysaccharide sur lequel sont fixés de façon statistique des groupes choisis parmi $-CH_2COOR^1$, $-CH_2-CO-Y$ ou $-CH_2-CO-NH-X$.

3. Objet selon la revendication 2, caractérisé en ce qu'il comprend des groupes $-CH_2-COOR^1$,

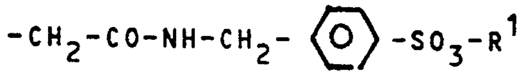 et 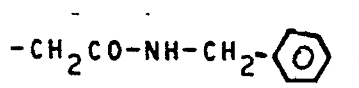 .

4. Objet selon la revendication 2, caractérisé en ce qu'il comprend des groupes $-CH_2-COOR^1$.

5. Objet selon la revendication 1, caractérisé en ce que le polymère est du polystyrène comportant des groupes choisis parmi les groupes de formule $-SO_3R^1$, $-CH_2-CO-NH-X$, $-COOR^1$,

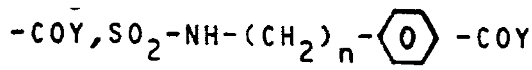

et

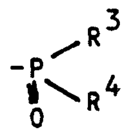

dans lesquelles $R^1$, $R^3$, $R^4$, X et Y ont la signification donnée dans la revendication 1.

6. Objet selon la revendication 1, caractérisé en ce que le polymère est du polystyrène comportant des groupes -COONa.

7. Objet selon la revendication 1, caractérisé en ce qu'il est réalisé au moins en partie en polystyrène comportant des groupes $-SO_3Na$.

8. Membrane de dialyse rénale caractérisée en ce qu'elle est réalisée au moins en partie en polysaccharide comportant dans sa chaîne des groupes substituables sur lesquels sont fixés de façon statistique des groupes inhibiteurs de l'activation du système du complément choisis parmi les groupes de formules $-CH_2COOR^1$, $-CH_2-CO-Y$ et $-CH_2-CO-NH-X$ dans lesquelles $R^1$ représente un atome d'hydrogène ou d'un métal physiologiquement acceptable, Y représente un radical dérivé d'un acide aminé lié au pont CO par sa fonction amine et X représente $-R^2-(SO_3)_p-R^1$ avec $p=0$ ou 1, $R^1$ représentant un atome d'hydrogène ou d'un métal physiologiquement acceptable, à condition que $R^1$ représente un atome d'hydrogène lorsque $p=0$ et $R^2$ représentant

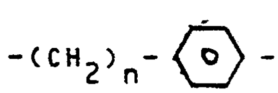

avec n étant un nombre entier de 1 à 4, ou un radical alkylène, arylène ou alkylènearylène substitué ou non.

9. Membrane selon la revendication 8, caractérisée en ce que le polysaccharide est la cellulose et en ce que les groupes inhibiteurs sont les groupes $-CH_2-COOR^1$ avec $R^1$ représentant un atome d'hydrogène ou d'un métal physiologiquement acceptable.

**10.** Sonde à usage médical ou chirurgical caractérisée en ce qu'elle est réalisée en polymère ou copolymère et en ce que sa surface destinée à venir en contact avec l'organisme est modifiée par greffage de polystyrène sur ledit polymère ou copolymère et fixation sur le polystyrène greffé de groupes de formules $-SO_2Y$, $-SO_3R^1$, $-CH_2-CO-NH-X$, $-COOR^1$, $-COY$,

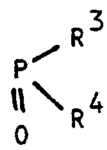 ,

$-CH_2-CO-NHR^2-SO_2-Y$, et/ou

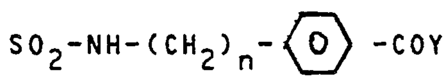

dans lesquelles $R^1$ représente un atome d'hydrogène ou d'un métal physiologiquement acceptable, $R^3$ et $R^4$ qui peuvent être identiques ou différents représentent $OR^1$ ou Y, Y représente un radical dérivé d'un acide aminé lié au pont $-SO_2-$, -CO- ou -PO- par sa fonction amine, X représente $R^2-(SO_3)_p-R^1$ avec p=0 ou 1, $R^1$ représentant un atome d'hydrogène ou d'un métal physiologiquement acceptable à condition que $R^1$ représente un atome d'hydrogène lorsque p=0, et $R^2$ représentant

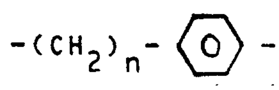

avec n étant un nombre entier de 1 à 4 ou un radical alkylène, arylène ou alkylènearylène substitué ou non et n est un nombre entier de 1 à 4.

**11.** Sonde selon la revendication 10, caractérisée en ce que le polymère est du polyéthylène.

**12.** Support pour chromatographie d'affinité ou pour épuration plasmatique n'entraînant pas une activation du système du complément, caractérisé en ce qu'il est constitué de grains de silice dont la surface est recouverte d'un polymère comportant dans sa chaîne des groupes substituables sur lesquels sont fixés de façon statistique des groupes inhibiteurs de l'activation du système du complément choisis parmi les groupes répondant aux formules suivantes :

1) - $-(CH_2)_mCOOR^1$, dans laquelle $R^1$ représente un atome d'hydrogène ou d'un métal physiologiquement acceptable et m est égal à 0 ou est un nombre entier de 1 à 15;

2) - $-CH_2-CO-NH-X$, dans laquelle X représente $R^2-(SO_3)_pR^1$ avec p étant égal à 0 ou 1, $R^1$ représentant un atome d'hydrogène ou d'un métal physiologiquement acceptable à condition que $R^1$ représente un atome d'hydrogène lorsque p est égal à 0, et $R^2$ représentant

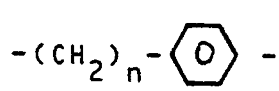

avec n étant un nombre entier de 1 à 4, ou un radical alkylène, arylène ou alkylènearylène substitué ou non ;

3) - $-CH_2-CO-NH-R^2-SO_2-Y$, dans laquelle $R^2$ représente

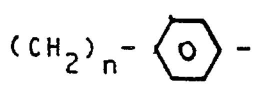

avec n étant un nombre entier de 1 à 4, ou un radical alkylène, arylène ou alkylènearylène substitué ou non, et Y représente un radical dérivé d'un acide aminé lié au pont $-SO_2-$ par sa fonction amine ;

4) - $-SO_2Y$ dans laquelle Y a la signification donnée ci-dessus,

5) - $-(CH_2)_mCOY$, dans laquelle Y représente un radical dérivé d'un acide aminé lié au pont -CO- par sa fonction amine, et m est égal à 0 ou est un nombre entier de 1 à 15,
6) - $-SO_3R^1$, dans laquelle $R^1$ a la signification donnée ci-dessus,
7) -

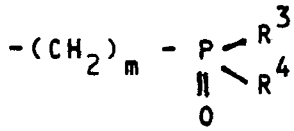

dans laquelle $R^3$ et $R^4$ qui peuvent être identiques ou différents, représentent $OR^1$ ou Y avec $R^1$ ayant la signification donnée ci-dessus et Y représentant un radical dérivé d'un acide aminé lié au pont -PO- par sa fonction amine, et m est égal à 0 ou est un nombre entier allant de 1 à 15, et
8) -

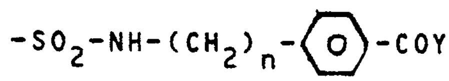

dans laquelle Y représente un radical dérivé d'un acide aminé lié au pont CO par sa fonction amine et n est un nombre entier de 1 à 4.

**13.** Support selon la revendication 12, caractérisé en ce que le polymère est du dextrane réticulé.

**14.** Support selon l'une quelconque des revendications 12 et 13, caractérisé en ce que les groupes inhibiteurs de l'activation du système du complément sont choisis parmi les groupes de formule $-CH_2COOR^1$, $-CH_2-CO-Y$ et $-CH_2-CO-NH-X$.

**15.** Utilisation, de polymères ou copolymères comportant dans leur chaîne des groupes substituables sur lesquels sont fixés de façon statistique des groupes inhibiteurs de l'activation du système du complément répondant à la formule :

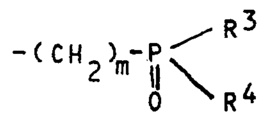

dans laquelle $R^3$ et $R^4$ qui peuvent être identiques ou différents, représentent $OR^1$ ou Y avec $R^1$ représentant un atome d'hydrogène ou d'un métal physiologiquement acceptable et Y représentant un radical dérivé d"un acide aminé lié au pont P=0 par sa fonction amine, et m est égal à 0 ou est un nombre entier allant de 1 à 15, pour la fabrication d'objets à usage médical ou chirurgical destinés à traiter ou réduire l'inflammation.

**16.** Utilisation selon la revendication 15, caractérisé en ce que $R^3$ représente ONa, R représente OH et m est égal à 0.

## Claims

**1.** Insoluble object for medical and/or surgical use, characterized in that it is at least partly made from a polymer or copolymer having in its chain substitutable groups to which are statistically fixed groups for inhibiting the activation of the complement system chosen from among groups complying with the following formulas:
1) $-(CH_2)_mCOOR^1$, in which $R^1$ represents a hydrogen atom or a physiologically acceptable metal and m is equal to 0 or is an integer between 1 and 15;
2) $-CH_2-CO-NH-X$ in which X represents $-R^2-H$, in which R" represents

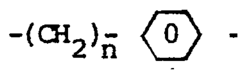

with n being an integer between 1 and 4 or a substituted or unsubstituted alkylene, arylene or alkylenearylene radical;
3) $-(CH_2)_mCOY$, in which Y represents a radical derived from an amino acid or an amino acid salt linked with the -CO- bridge by its amine function and m is equal to 0 or is an integer from 1 to 15;
4) $-SO_3R^1$, in which $R^1$ has the meaning given hereinbefore;
5)

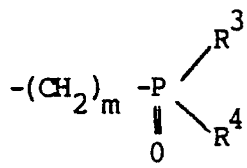

in which $R^3$ and $R^4$, which can be the same or different, represent $OR^1$ or Y with $R^1$ having the meaning given hereinbefore, Y representing a radical derived from an amino acid linked with the -PO- bridge by its amine function and m being equal to 0 or an integer between 1 and 15, provided that $R^3$ and $R^4$ represent OH or Y when m is equal to 0; and
6)

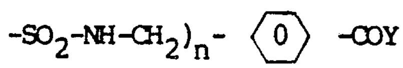

in which Y represents a radical derived from an amino acid linked with the CO bridge by its amine function and n is an integer from 1 to 4, provided that the polymer or copolymer only has a single type of inhibiting group when such groups have the formula $-SO_3R^1$ or $-(CH_2)_mCOY$ with m being equal to 1.

2. Object according to claim 1, characterized in that the polymer is a polysaccharide to which are statistically fixed groups chosen from among $-CH_2COOR^1$, $-CH_2-CO-Y$ or $-CH_2-CO-NH-X$.

3. Object according to claim 2, characterized in that it comprises the groups

$-CH_2-COOR^1$, 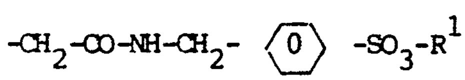 and 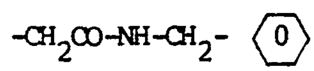 .

4. Object according to claim 2, characterized in that it comprises $-CH_2-COOR^1$ groups.

5. Object according to claim 1, characterized in that the polymer is polystyrene having groups chosen from among the groups of formula $-SO_3R^1$, $-CH_2-CO-NH-X$, $-COOR^1$,

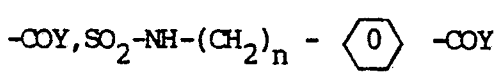

and

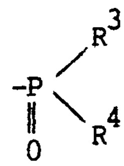

in which $R^1$, $R^3$, $R^4$, X and Y have the meanings given in claim 1.

**6.** Object according to claim 1, characterized in that the polymer is polystyrene having -COONa groups.

**7.** Object according to claim 1, characterized in that it is at least partly made from polystyrene having $-SO_3Na$ groups.

**8.** Renal dialysis membrane, characterized in that it is at least partly produced from a polysaccharide having in its chain substitutable groups to which are statistically fixed groups inhibiting the activation of the complement system and chosen from among the groups of formula $-CH_2COOR^1$, $-CH_2-CO-Y$ and $-CH_2-CO-NH-X$ in which $R^1$ represents a hydrogen atom or a physiologically acceptable metal, Y represents a radical derived from an amino acid linked with the CO bridge by its amine function and X represents $-R^2-(SO_3)_p-R^1$ with $p=0$ or 1, $R^1$ representing a hydrogen atom or a physiologically acceptable metal, provided that $R^1$ represents a hydrogen atom when $p=0$ and $R^2$ representing

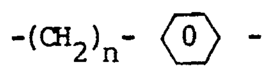

with n being an integer from 1 to 4 or a substituted or unsubstituted alkylene, arylene or alkylenearylene radical.

**9.** Membrane according to claim 8, characterized in that the polysaccharide is cellulose and wherein the inhibiting groups are $-CH_2COOR^1$ groups with $R^1$ representing a hydrogen atom or a physiologically acceptable metal.

**10.** Probe for medical or surgical use, characterized in that it is made from a polymer or copolymer and in that its surface to come into contact with the organism is modified by grafting polystyrene onto said polymer or copolymer and fixing to the grafted polystyrene groups of formula $-SO_2Y$, $-SO_3R^1$, $-CH_2-CO-NH-X$, $-COOR^1$, -COY,

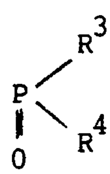 ,

$-CH_2-CO-NHR^2-SO_2-Y$, and/or

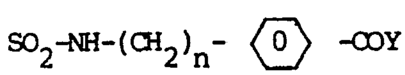

in which $R^1$ represents a hydrogen atom or a physiologically acceptable metal, $R^3$ and $R^4$, which can be the same or different, represent OR or Y, Y represents a radical derived from an amino acid linked with the bridge $-SO_2-$, -CO- or -PO- by its amino function, X represents $R^2-(SO_3)_p-R^1$ with $p=0$ or 1, $R^1$ represents a hydrogen atom or a physiologically acceptable metal, provided that $R^1$ represents a hydrogen atom when $p=0$ and $R^2$ represents

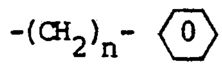

with n being an integer from 1 to 4 or a substituted or unsubstituted alkylene, arylene or alkylenearylene radical and n is an integer from 1 to 4.

**11.** Probe according to claim 10, characterized in that the polymer is polyethylene.

**12.** Support for affinity chromatography or plasma purification not bringing about any activation of the complement system, characterized in that it is constituted by silica grains, whose surface is covered with a polymer having in its chain substitutable groups, to which are statistically fixed groups inhibiting the activation of the complement system chosen from among the groups complying with the following formulas:

1) $-(CH_2)_mCOOR^1$, in which $R^1$ represents a hydrogen atom or a physiologically acceptable metal and m is equal to 0 or is an integer from 1 to 15;

2) $-CH_2-CO-NH-X$, in which X represents $R^2-(SO_3)_{\bar{p}}R^1$ with p being equal to 0 or 1 and $R^1$ representing a hydrogen atom or a physiologically acceptable metal, provided that $R^1$ represents a hydrogen atom when p is equal to 0 and $R^2$ represents

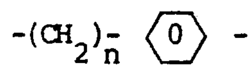

with n being an integer from 1 to 4 or a substituted or unsubstituted alkylene, arylene or alkylenearylene radical;

3) $-CH_2-CO-NH-R^2-SO_2-Y$, in which $R^2$ represents

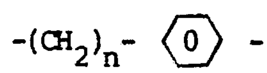

with n being an integer from 1 to 4 or a substituted or unsubstituted alkylene, arylene, or alkylenearylene radical and Y represents a radical derived from an amino acid or an amino acid salt linked with the $-SO_2-$ bridge by its amine function;

4) $-SO_2Y$, in which Y has the meaning given hereinbefore;

5) $-(CH_2)_mCOY$, in which Y represents a radical derived from an amino acid linked with the -CO- bridge by its amine function and m is equal to 0 or is an integer from 1 to 15;

6) $-SO_3R^1$ in which $R^1$ has the meaning given hereinbefore;

7)

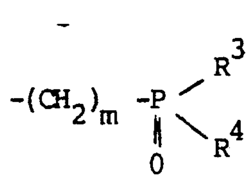

in which $R^3$ and $R^4$, which can be the same or different, represent $OR^1$ or Y with $R^1$ having the meaning given hereinbefore and Y representing a radical derived from an amino acid linked with the -PO- bridge by its amine function and m being equal to 0 or being an integer from 1 to 15; and

8)

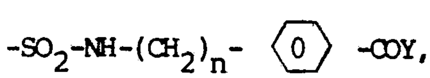

in which Y represents a radical derived from an amino acid linked with the CO bridge by the amine function and n is an integer from 1 to 4.

**13.** Support according to claim 12, characterized in that the polymer is crosslinked dextran.

**14.** Support according to either of the claims 12 and 13, characterized in that the groups inhibiting the activation of the complement system are chosen from among the groups of formula $-CH_2COOR^1$, $-CH_2-CO-Y$ and $-CH_2-CO-NH-X$.

**15.** Use of polymers or copolymers having in their chain substitutable groups to which are statistically fixed groups inhibiting the activation of the complement system according to formula:

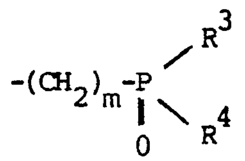

in which $R^3$ and $R^4$, which can be the same or different, represent $OR^1$ or Y with $R^1$ representing a hydrogen atom or a physiologically acceptable metal and Y represents a radical derived from an amino acid linked to the P=0 bridge by its amine function and m is equal to 0 or is an integer from 1 to 15, for the production of objects for medical or surgical use intended for the treatment or reduction of inflammation.

**16.** Use according to claim 15, characterized in that $R^3$ represents ONa, $R^4$ represents OH and m is equal to 0.

**Patentansprüche**

**1.** Unlöslicher Gegenstand für die medizinische oder chirurgische Verwendung, dadurch gekennzeichnet, daß er mindestens zum Teil hergestellt ist aus einem Polymeren oder Copolymeren, das in seiner Kette substituierbare Gruppen aufweist, an denen in statistischer Weise Inhibitorgruppen der Aktivierung des Komplement- Systems fixiert sind, die ausgewählt werden aus Gruppen der nachstehend angegebenen Formeln:

(1) $-(CH_2)_mCOOR^1$, worin $R^1$ ein Wasserstoffatom oder ein physiologisch akzeptables Metall und m die Zahl 0 oder eine ganze Zahl von 1 bis 15 bedeuten,

(2) $-CH_2-CO-NH-X$, worin X $-R^2-H$, worin $R^2$ steht für

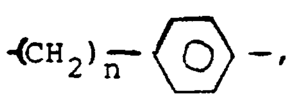

worin n eine ganze Zahl von 1 bis 4 darstellt, oder einen substituierten oder unsubstituierten Alkylen-, Arylen- oder Alkylenarylenrest bedeutet,

(3) $-(CH_2)_m-CO-Y$, worin Y einen Rest darstellt, der von einer Aminosäure oder einem Salz einer Aminosäure abgeleitet ist, der über seine Aminfunktion an die -CO-Brücke gebunden ist, und m die Zahl 0 oder eine ganze Zahl von 1 bis 15 bedeuten,

(4) $-SO_3R^1$ , worin $R^1$ die oben angegebene Bedeutung hat,

(5)

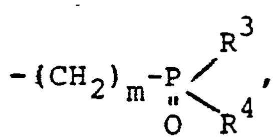

worin $R^3$ und $R^4$, die gleich oder verschieden sein können, $OR^1$ oder Y bedeuten, worin $R^1$ die oben angegebene Bedeutung hat und Y einen Rest darstellt, der von einer Aminosäure abgeleitet ist, der über seine Aminfunktion an die -CO-Brücke gebunden ist, und m die Zahl 0 oder eine ganze Zahl von 1 bis 15 bedeutet, mit der Maßgabe, daß $R^3$ und $R^4$ OH oder y darstellen, wenn m für die Zahl 0 steht, und

(6)

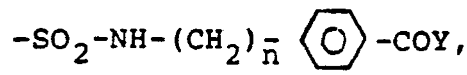

worin Y einen Rest darstellt, der von einer Aminosäure abgeleitet ist, der über die Aminfunktion an die CO-Brücke gebunden ist, und n eine ganze Zahl von 1 bis 4 bedeutet,

mit der Maßgabe, daß das Polymere oder das Copolymere nur einen einzigen Typ von Inhibitor-Gruppen aufweist, wenn diese Gruppen die Formel $-SO_3R^1$ oder $-(CH_2)_mCOY$ mit m = 1 haben.

2. Gegenstand nach Anspruch 1, dadurch gekennzeichnet, daß das Polymere ein Polysaccharid ist, an dem auf statistische Weise Gruppen, ausgewählt aus $-CH_2COOR^1$, $-CH_2$-CO-Y- oder $-CH_2$-CO-NH-X-Gruppen,fixiert sind.

3. Gegenstand nach Anspruch 2, dadurch gekennzeichnet, daß er $-CH_2-COOR^1$-,

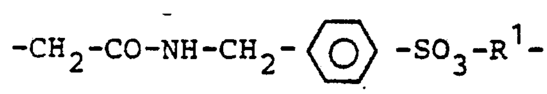

und

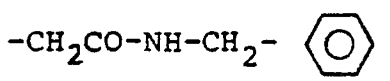

Gruppen aufweist.

4. Gegenstand nach Anspruch 2, dadurch gekennzeichnet, daß er $-CH_2-COOR^1$-Gruppen aufweist.

5. Gegenstand nach Anspruch 1, dadurch gekennzeichnet, daß das Polymere Polystyrol ist, das Gruppen aufweist, die ausgewählt werden aus den Gruppen der Formeln $-SO_3R^1$, $-CH_2$-CO-NH-X, $-COOR^1$,

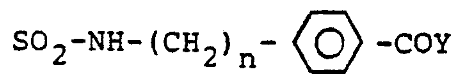

und

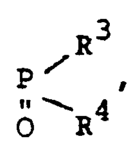

worin $R^1$, $R^3$, $R^4$, X und Y die in Anspruch 1 angegebenen bedeutungen haben.

6. Gegenstand nach Anspruch 1, dadurch gekennzeichnet, daß das Polymere Polystyrol ist, das -COONa-Gruppen aufweist.

7. Gegenstand nach Anspruch 1, dadurch gekennzeichnet, daß er mindestens zum Teil aus Polystyrol hergestellt ist, das $-SO_3Na$-Gruppen aufweist.

8. Nierendialysemembran, dadurch gekennzeichnet, daß sie mindestens zum Teil aus einem Polysaccharid hergestellt ist, das in seiner Kette substituierbare Gruppen aufweist, an denen in statistischer Weise Inhibitorgruppen der Aktivierung des Komplementsystems fixiert sind, die ausgewählt werden aus Gruppen der Formeln $-CH_2COOR^1$, $-CH_2$-CO-Y und $-CH_2$-CO-NH-X, worin $R^1$ ein Wasserstoffatom oder

ein physiologisch akzeptables Metall, Y einen Rest, der von einer Aminosäure abgeleitet ist, der über seine Aminfunktion an die CO-Brücke gebunden ist, und X $-R^2-(SO_3)_p-R^1$ bedeuten, worin p = 0 oder 1, $R^1$ ein Wasserstoffatom oder ein physiologisch akzeptables Metall, mit der Maßgabe, daß $R^1$ ein Wasserstoffatom darstellt, wenn p = 0, und $R^2$

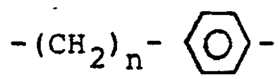

mit n = eine ganze Zahl von 1 bis 4 oder einen substituierten oder unsubstituierten Alkylen-, Arylen- oder Alkylenarylenrest bedeuten.

**9.** Membran nach Anspruch 8, dadurch gekennzeichnet, daß das Polysaccharid Cellulose ist und daß die Inhibitorgruppen $-CH_2-COOR^1$ -Gruppen sind, in denen $R^1$ ein Wasserstoffatom oder ein physiologisch akzeptables Metall bedeutet.

**10.** Sonde für die medizinische oder chirurgische Verwendung, dadurch gekennzeichnet, daß sie hergestellt ist aus einem Polymeren oder Copolymeren und daß ihre Oberfläche, die mit dem Organismus in Kontakt kommen soll, modifiziert worden ist durch Aufpfropfen von Polystyrol auf das Polymere oder Copolymere und durch Fixieren an dem aufgepfropften Polystyrol von Gruppen der Formeln $-SO_2Y$, $-SO_3R^1$, $-CH_2-CO-NH-X$, $-COOR^1$, $-COY$,

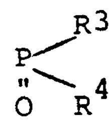
,

$-CH_2-CO-NHR^2-SO_2-Y$ und/oder

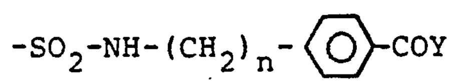
,

worin $R^1$ ein Wasserstoffatom oder ein physiologisch akzeptables Metall, $R^3$ und $R^4$, die gleich oder verschieden sein können, $OR^1$ oder Y, worin Y einen Rest darstellt, der von einer Aminosäure abgeleitet ist, der über seine Aminfunktion an die $-SO_2-$, -CO- oder -PO-Brücke gebunden ist, X $R^2-(SO_3)_pR^1$, worin p = 0 oder 1 und $R^1$ ein Wasserstoffatom oder ein physiologisch akzeptables Metall darstellen, mit der Maßgabe, daß $R^1$ ein Wasserstoffatom darstellt, wenn p = 0, und $R^2$

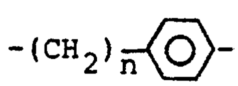

darstellt, worin n für eine ganze Zahl von 1 bis 4 steht, oder einen substituierten oder unsubstituierten Alkylen-, Arylen-oder Alkylenarylenrest und n eine ganze Zahl von 1 bis 4 bedeuten.

**11.** Sonde nach Anspruch 10, dadurch gekennzeichnet, daß das Polymere Polyethylen ist.

**12.** Träger für die Affinitätschromatographie oder für die Plasmareinigung, der keine Aktivierung des Komplement-Systems mit sich bringt, dadurch gekennzeichnet, daß er besteht aus Siliciumdioxid-Körnchen, deren Oberfläche von einem Polymeren bedeckt ist, das in seiner Kette substituierbare Gruppen aufweist, an denen auf statistische Weise Inhibitorgruppen der Aktivierung des Komplement-Systems fixiert sind, die ausgewählt werden aus Gruppen der folgenden Formeln:

(1) $-(CH_2)_mCOOR^1$, worin $R^1$ ein Wasserstoffatom oder ein physiologisch akzeptables Metall und m die Zahl 0 oder eine ganze Zahl von 1 bis 15 bedeuten,

(2) $-CH_2-CO-NH-X$, worin X $R^2-(SO_3)_p-R^1$ bedeutet, worin p für die Zahl 0 oder 1, $R^1$ für ein Wasserstoffatom oder ein physiologisch akzeptables Metall mit der Maßgabe, daß $R^1$ ein Wasserstoffatom darstellt, wenn p = 0, und $R^2$ für

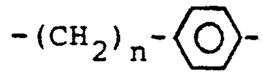

stehen, worin n eine ganze Zahl von 1 bis 4 darstellt, oder einen substituierten oder unsubstituierten Alkylen-, Arylen- oder Alkylenarylenrest bedeutet;
(3) $-CH_2-CO-NH-R^2-SO_2-Y$, worin Y

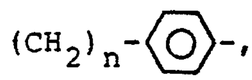

worin n eine ganze Zahl von 1 bis 4 darstellt, oder einen substituierten oder unsubstituierten Alkylen-, Arylen- oder Alkylenarylenrest und Y einen Rest bedeuten, der von einer Aminosäure abgeleitet ist, der über seine Aminfunktion an eine $-SO_2$-Brücke gebunden ist;
(4) $-SO_2Y$, worin Y die oben angegebene Bedeutung hat;
(5) $-(CH_2)_mCOY$, worin Y einen Rest, der von einer Aminosäure abgeleitet ist, die über ihre Aminfunktion an die -CO-Brücke gebunden ist, und m die Zahl 0 oder eine ganze Zahl von 1 bis 15 bedeuten;
(6) $-SO_3R^1$, worin $R^1$ die oben angegebene Bedeutung hat;
(7)

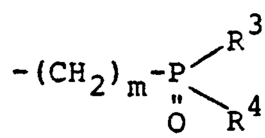

worin $R^3$ und $R^4$, die gleich oder verschieden sein können, $OR^1$ oder Y bedeuten, worin $R^1$ die oben angegebene Bedeutung hat und y einen Rest darstellt, der von einer Aminosäure abgeleitet ist, der über seine Aminfunktion an eine -CO-Brücke gebunden ist, und m die Zahl 0 oder eine ganze Zahl von 1 bis 15 bedeutet; und
(8)

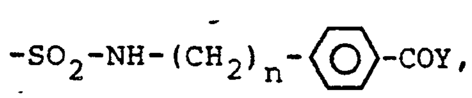

worin Y einen Rest darstellt, der von einer Aminosäure abgeleitet ist, die über ihre Aminfunktion an die -CO-Brücke gebunden ist, und n eine ganze Zahl von 1 bis 4 bedeutet.

**13.** Träger nach Anspruch 12, dadurch gekennzeichnet, daß das Polymere vernetztes Dextran ist.

**14.** Träger nach einem der Ansprüche 12 und 13, dadurch gekennzeichnet, daß die Inhibitorgruppen der Aktivierung des Komplementsystems ausgewählt werden aus den Gruppen der Formeln $CH_2COOR^1$, $-CH_2-CO-Y$ und $-CH_2-CO-NH-X$.

**15.** Verwendung von Polymeren oder Copolymeren, die in ihrer Kette substituierbare Gruppen aufweisen, an denen in statistischer Weise Inhibitorgruppen der Aktivierung des Komplementsystems fixiert sind, die der Formel entsprechen

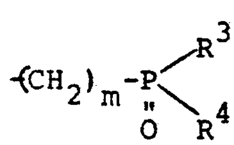

worin $R^3$ und $R^4$, die gleich oder verschieden sein können, $OR^1$ oder Y bedeuten, worin $R^1$ ein

Wasserstoffatom oder ein physiologisch akzeptables Metall und Y einen Rest darstellen, der von einer Aminosäure abgeleitet ist, der über seine Aminfunktion an eine -P=O-Brücke gebunden ist, und m die Zahl 0 oder eine ganze Zahl von 1 bis 15 bedeutet,
für die Herstellung von Gegenständen für die medizinische oder chirurgische Verwendung, die dafür bestimmt sind, eine Entzündung zu behandeln oder zu lindern.

**16.** Verwendung nach Anspruch 15, dadurch gekennzeichnet, daß $R^3$ für ONa, $R^4$ für OH und m für die Zahl 0 stehen.

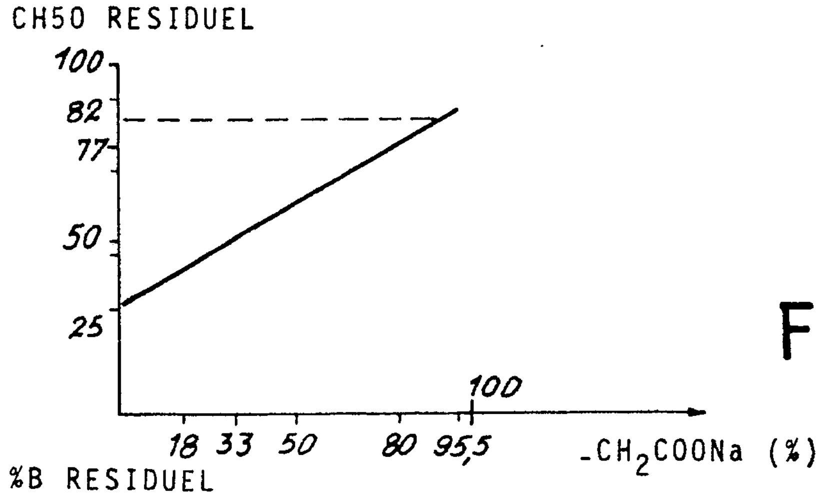


FIG.1

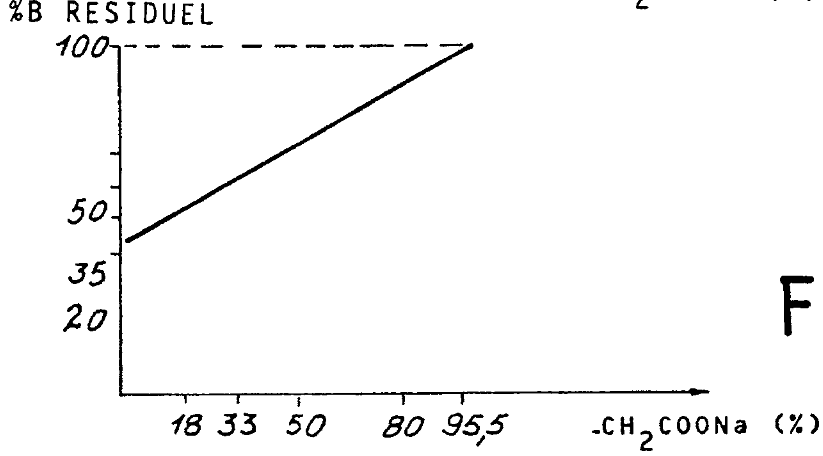


FIG.2

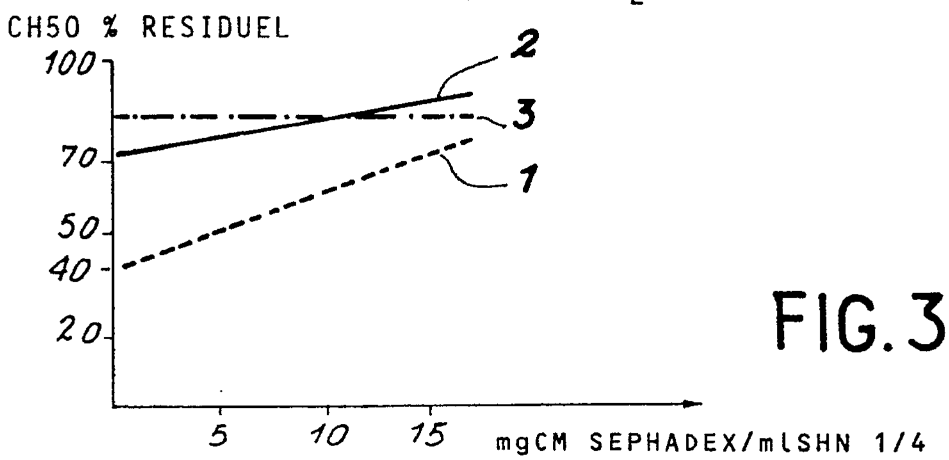


FIG.3

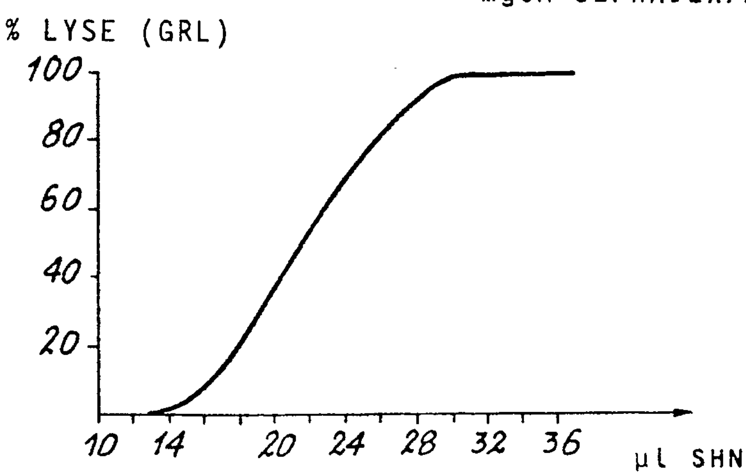


FIG.4

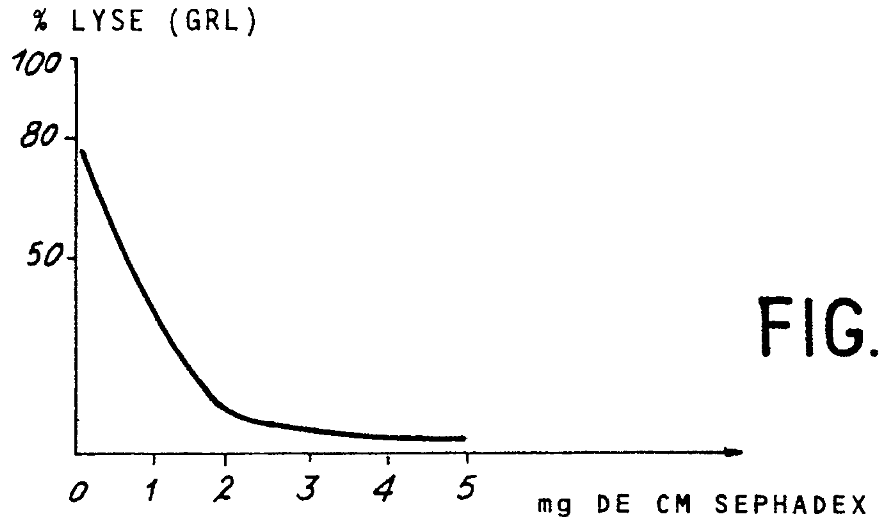


FIG.5

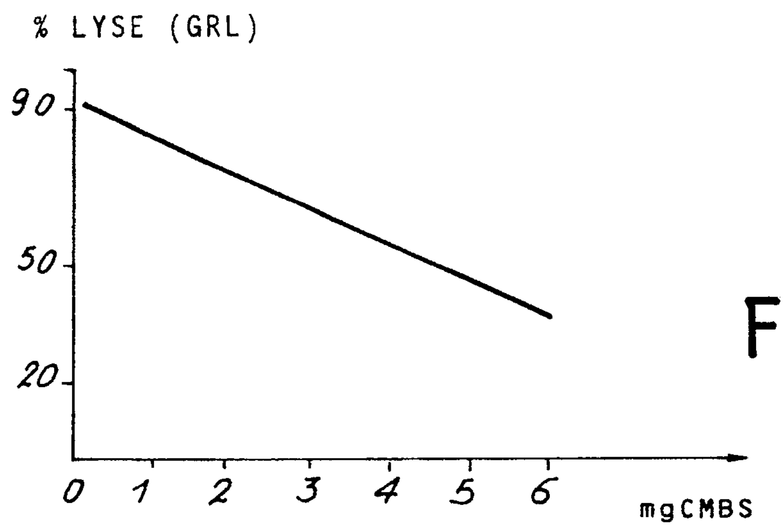


FIG.6

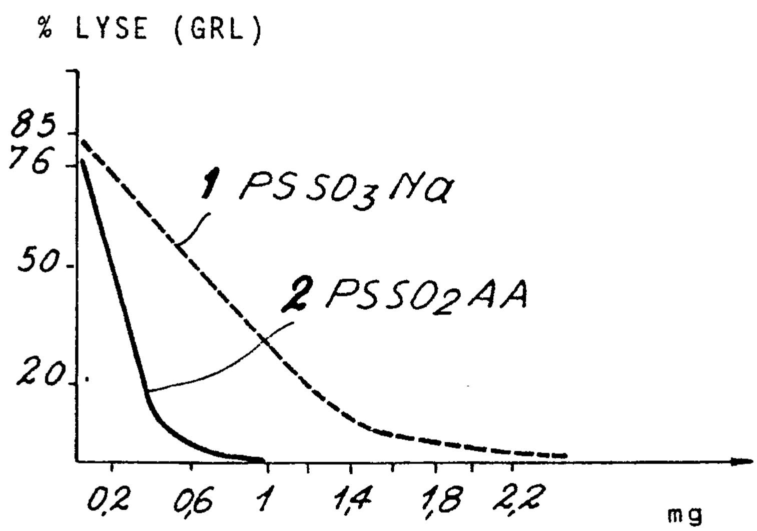


FIG.7

FIG.8